# EUROPEAN PATENT APPLICATION

(11) **EP 2 805 733 A1**
(43) Date of publication of application: **26.11.2014**
(21) Application number: 13739005.0
(22) Date of filing: 21.01.2013
(51) Int. Cl.: A61K 49/06, C12N 15/12, C12N 15/17, C07K 14/47

(54) **CELL-TARGETED MAGNETIC NANO-MATERIAL AND BIOMEDICAL USES THEREOF**

(30) Priority: 19.01.2012 CN 201210018276
(71) Applicant: Institute of Geology and Geophysics, Chinese Academy of Sciences, Beijing 100029 (CN)
(72) Inventor: PAN, Yongxin, Beijing 100029 (CN); CAO, Changqian, Beijing 100029 (CN); TIAN, Lanxiang, Beijing 100029 (CN); CAI, Yao, Beijing 100029 (CN); ZHU, Rixiang, Beijing 100029 (CN)
(74) Representative: Osha Liang SARL
(86) International application number: PCT/CN2013/070772
(87) International publication number: WO 2013/107415

(57) **Abstract**

Disclosed are a cell-targeted nano-material and biomedical uses thereof. The magnetic nano-material can bind with specificity to iron protein receptors having high expression on the surface of tissue cells, and can enter the cells. The present material can bind with specificity to a broad spectrum of tissue cells having a high expression of iron protein receptors, and can enable highly efficient cell targeting in animal models. The material can be used as a magnetic resonance imaging contrast and a fluorescent molecular probe for disease diagnosis, as well as a vector of medicine for disease treatment.

## Description

### TECHNICAL FIELD

The present invention relates to the interdisciplinary fields of biomimetic synthesis, nanotechnology, molecular imaging, and biomedicine, more particularly, to a cell-targeting magnetic nano-material and biomedical uses thereof.

### BACKGROUND

Magnetic Resonance Imaging (MRI) has the unique advantages of non-invasiveness and high resolution, and thus it is a very good imaging tool for use in early diagnosis of diseases. However, it has a low sensitivity, cannot well distinguish diseased tissues from healthy ones, and lacks specificity in detection. These are the inherent disadvantages of MRI [*Terreno et al.*, 2010]. To overcome the drawback of low sensitivity, researchers have tried to synthesize different kinds of MRI contrast agents and used them to shorten the longitudinal relaxation time (T₁) or the transverse relaxation time (T₂), in order to increase the sensitivity and tissue contrast of magnetic resonance. Based on different types of relaxation time, MRI contrast agents are sorted into T₁ contrast agent and T₂ contrast agent. By shortening T₁, T₁ contrast agent increases the signal intensity and brightens the target area in T₁-weighted image. Most of such contrast agents use the lanthanide element gadolinium or materials derived therefrom and these agents are most widely used as MRI contrast agents clinically. However, after being used clinically for nearly 30 years, gadolinium-based contrast agent exposes many defects, such as low sensitivity, short half-life and significant side effects. For example, US FDA issued two communications in 2007 and 2010 that gadolinium-based contrast agents cause patients with specific kidney disease to develop fatal nephrogenic systemic fibrosis (FDA news release, FDA Requests Boxed Warning for Contrast Agents Used to Improve MRI Images, 5/23/2007; FDA news release, FDA: New warnings required on use of gadolinium-based contrast agents, 9/9/2010). A T₂ contrast agent mainly comprises super paramagnetic iron oxide (SPIO) particles. When T₂ and T₂* weighed imaging series are used in the magnetic resonance imaging machine, the proton signal intensity of SPIO area is decreased, showing dark signals in the image. Compared with conventional gadolinium-based contrast agents, SPIO particles have the following advantages: (1) high sensitivity: every metal unit can change MRI signal intensity maximally, and especially, in T₂*-weighed image, signal to noise ratio (SNR) is increased significantly; (2) removablitily by metabolism in the organism: iron is metabolizable in the organism, and super paramagnetic magnetites can be utilized iron circulation of the organism; (3) they can be surface modified easily, and can be linked to different functional groups and ligands; (4) as they enter the organism, they can be observed by optical microscope and electron microscope; (5) the magnetic property and relaxation effect of the particles can be adjusted by changing grain size and shape through different chemical synthesis conditions. Therefore, SPIO particles are a promising MRI contrast agent [Bulte and Kraitchman, 2004].

In addition, magnetic nano-material has big specific surface area, high loading efficiency and high magnetic susceptibility. Thus, it is an excellent drug vector, which is able to lengthen the effective time of drugs, enhance the effect of drugs, decrease toxic and side effect, enhance the stability of drugs and protect the unstable drugs from degradation. Therefore, magnetic nano-material has become an ideal choice for highly efficient drug carrying system, and hopefully it could overcome the defects of conventional chemical drugs, such as large dosage, significant toxic and side effect, low efficiency and poor stability.

However, all the conventional nano-materials are synthesized by physicochemical methods. Although these methods have been developed for many years, yet none of them could produce the magnetic nano-material that has uniform grain size and shape, high dispersity, hydrophilicity and high biocompatibility simultaneously. Generally, the synthesis of magnetic nano-material demands intense physicochemical conditions (high temperature, high pressure, and high pH). After being synthesized, the magnetic nano-material needs expensive and complicated surface modification so as to ensure its high dispersity, hydrophilicity and biocompatibility. Moreover, regarding the medical application of magnetic nano-materials, the physicochemically synthesized magnetic nano-material does not target specific cells, and tends to be phagocytized by reticuloendothelia system (RES) after it enters the body. In order to realize specific cell targeting ability in vivo, the conventional magnetic nano-materials have to be surface modified complicatedly and linked to specific ligands that target specific cells (for instance, tumor cells), such as antibodies, peptide fragments, targeting small molecules etc. [*Harisinghani et al.*, 2003; *Lin et al.*, 2010; *McCarthy et al.*, 2007]. Nevertheless, the conventional magnetic nano-materials produced through chemical surface modification and coupling targeting ligands have many disadvantages: (1) the steps of modification and ligands linking are complicated, and there is difficulty in quantification and ensuring uniformity; (2) after modification, the magnetic nano-materials tend to change features such as grain sizes and surface properties; (3) their targeting affinity to lesion tissues is low and massive materials are still phagocytized by reticuloendothelia system (the major disadvantage of this kind of material); (4) besides, after the magnetic nano-materials enter the body, there is still a problem in stability. Take the one linked to targeting polypeptides on the surface for example, after it enters the body, it tends to be adsorbed without specificity and degraded by protease. Generally, it needs to be linked to multiple targeting molecules on the surface or adding D-amino acid to prevent from degradation, and thus cannot be used in early diagnosis and treatment [*Byrne et al.*, 2008; *Lee et al.*, 2007; *McCarthy et al.*, 2010].

When we encounter problems such as high energy consumption and difficulty in modification in employing conventional physicochemical synthesis of magnetic nano-materials, biomineralization of nature and biomimetic synthesis provide us with a new idea. Biomineralization is a process that inorganic minerals are formed under the control of genes or biomacromolecules under physiological conditions. Based on biomineralization mechanism, biomimetic synthesis is a synthesizing process of inorganic materials under the control of organics, which simulates the formation process of inorganics in biomineralization, so as to obtain materials with uniform size , specific assembly structure and specific biomacromolecules coating. Biomimetic synthesis can produce a magnetic nano-material having uniform grain size, high dispersity, hydrophilicity and high biocompatibility with simple steps and low energy consumption. Therein, ferritins and magnetotactic bacteria Mms6 are typical superparamagnetic materials by biomimetic synthesis.

Ferritins are an important iron-storage proteins that participate in and maintain the iron metabolism in the organism, and widely exist in the cells of animals, plants and microorganisms. They have typical core-shell nanostructures: the core is ferric oxide hydrate particles (6-8 nm), and the shell is a cage-shaped protein shell (12nm) self-assembled by 24 protein subunits. The formation of iron protein shells is highly controlled by the genes of organism. They have highly uniform grain size and shape, and thus provide natural bionanoreactors for biomimetic preparation of SPIO particles [*Harrison and Arosio,* 1996; *Uchida et al*. 2007].More importantly, many fast-growing cells (like tumor cells) need a great quantity of iron nutrients, and highly express ferritin receptors. Fargion et al. used human proerythroleukemic cells K562, HL-60 cell line, small-cell lung cancer cells NCI-N417 to bind to human ferritin composed solely of heavy (H) chain subunits, and human ferritin composed solely of light (L) chain subunits and the hybrid ferritin thereof. The result shows that K562 cells can bind with specificity to human ferritin composed solely of H-subunits and that composed of H and L chain subunits. The affinity constant of the binding to human ferritin is as high as 3×10⁸M⁻¹ [*Fargion et al.*, 1988]. Moss et al. studied specific human H-subunit ferritin receptors of highly proliferative tumor cells and normal cells, and found that the tumor cells highly express specific human H-subunit ferritins receptors, while such expression is hardly detected on normal cells [*Moss et al.*, 1992]. Recently, Li et al. found: human transferrin receptor-1 (TfR1) is a co-receptor for human H-subunit ferritin and transferrin, which can bind with specificity to human H-subunit ferritin, and endocytose it into endosomes and lysosomes [*Li et al.*, 2010]. During an inflammatory process, the organism secretes a large amount of inflammatory cytokines to stimulate inflammatory cells to highly express ferritin receptors, and thus maintains iron supply [*Fahmy and Young*, 1993; *Byrd and Horwitz,* 2007].

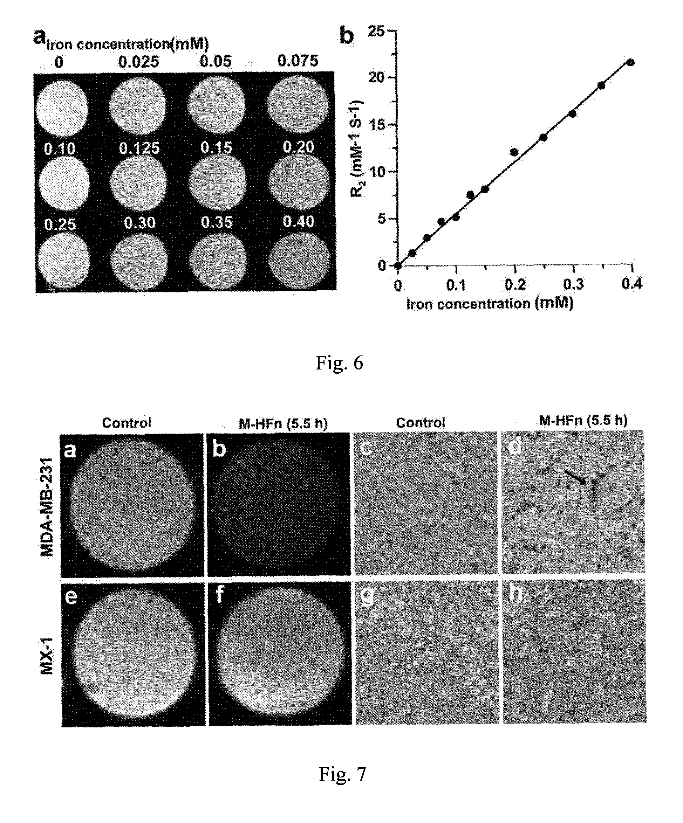

The core of natural ferritin is a ferric oxide hydrate with low magnetic susceptibility (weak magnetism) and low relaxation effect, which considerably limits its application in biomedicine as a magnetic nano-material directly. The key to solving the application problem of ferritin is to turn the weak magnetic core into a strong one (like magnetite and/or maghemite). Magnetoferritin with a strongly magnetic magnetite core was firstly synthesized by Meldrum et al. in 1992. But due to the immaturity of the synthesis process, much iron deposits on the surface of protein shells. This kind of ferritin not only tends to aggregate, but also has no tumor targeting ability after entering human body, and could be easily phagocytized by reticuloendothelia system [*Meldrum et al.*, 1992; *Moskowitz et al.*, 1997; *Bulte et al.*, 1995].The latest international development is that, Uchida et al. used genetically engineered or RGD incorporated human H-subunit ferritins as templates and thus biomimetically synthesized magnetoferritins with cores having magnetite or maghemite cores. But the hysteresis loop data shows that, the synthesized magnetoferritins cannot reach saturation at 80000 Gauss (8T) (common ferromagnetic magnetite or hematite is saturated below IT). This indicates that, due to the limitation of synthesis process, the composition of synthesized magnetoferritins is complex, containing not only ferromagnetic magnetite or maghemite, but also antiferromagnetic or paramagnetic composition [Uchida et al., 2006, Figure 7].In addition, in vivo and in vitro experiments showed that, in vivo application of the material is still the same as that of magnetic nano-materials synthesized by ordinary chemical process, which is to diagnose the diseases showing an abnormal increase of macrophages. For example, Terashima et al. used the magnetoferritins to experiment on animal models with atherosclerosis. They found that the material can be phagocytized by macrophages in vessels, and thus can be used as a magnetic resonance contrast agent in the diagnosis of atherosclerosis [Uchida et al., 2008; Tetashima et al., 2011].Up till now, there is no report about the use of magnetoferritins in targeting diagnosis of tumor. Based on previous synthesis methods for magnetoferritins, our lab directly used the empty shell human H-subunit ferritins expressed by genetic engineering directly as nanoreactors in 2010, whereby core-shell magnetic nano-material completely coated with ferritins can be formed through only one step of biomimetic mineralization reaction. The material has properties such as uniform grain size, uniform shape, monodispersity, water solubility etc. The synthesis of magnetoferritins with human source provides a new idea for the use of biomimetic ferritin magnetic nano-materials in human body [*Cao et al.*, 2010; Chinese invention patent: 200910244505.1; 201010541069.7].

Magnetotactic bacteria are microorganisms that can move along the direction of magnetic field. They were found in the 1960s and 1970s, widely distributed in lakes, seas and even wet soils. To date, 10 new species of magnetotactic bacteria have been found in sediments in China [*lin et al.*, 2011]. Magnetotactic bacteria have a diversity of morphologies including coccidian, vibrion, helicobacter, bacillus, aggregates etc., and belong to proteobacteria and nitrospirae in phylogenesis. A common feature of these microorganisms is that they can in vivo synthesize nano-magnetite (30-120 nm) magnetosomes that are chain-like arranged, highly purified and coated with biological membranes. Magnetosomes synthesized by different species of magnetotactic bacteria have specific crystal shapes, namely cubo-octahedron, pseudohexagonal prism and bullet-head shape. As single magnetic domain materials coated with natural biological membranes, magnetosomes have broad prospect of development and application in the fields of biomedicine and material science [*Arakaki et al.*, 2008]. There are large quantities of membrane proteins on the surfaces of the magnetosomes of magnetotactic bacteria, and these membrane proteins control the formation of nano-magnetite. Mms6 protein is an important membrane protein of magnetotactic bacteria. It adsorbs onto the surface of the magnetosome tightly. Both in vitro and vivo experiments verified that Mms6 protein could adjust the shapes and sizes of magnetites. However, in the past, biomimetic synthesis overly depended on intense chemical conditions that might destabilize proteins (controlling the grain sizes at a temperature as high as 90 °C or by adding polymeric gel), and thus did not exploit magnetotatic bacteria's advantages of the ability to control the grain sizes, shapes and crystallinity at ambient temperature and pressure. Therefore, it is an urgent scientific problem that how the biomimetic synthesis process can be improved so as to realize the biomimetic synthesis of membrane proteins of magnetotatic bacteria [*Arakaki et al.*, 2003; *Arakaki et al.*, 2007; Prozorov et al., 2007; *Arakaki et al.*, 2010].

Usually, magnetic nano-materials are biomimetically synthesized by bio-proteins under low energy consuming conditions (low temperature, low pressure and low pH), and biomimetic process is highly efficient, ordered and self-assembled. The materials obtained therefrom have many advantages in material science, such as uniform grain size, uniform shape, high dispersity and hydrophilicity etc. The crux problem needs solving in the field is how to improve and use these biomineralization proteins to synthesize new magnetic nano-materials coated with proteins. Based on the ferritin shells' natural cells targeting ability that they can bind to the ferritin receptors highly expressed on the surface of highly proliferative cells, the synthesis method is improved to achieve the targeting ability, especially in vivo, of biomimetic protein magnetic nano-materials to the cells highly expressing ferritin receptors. The material synthesized therefrom can be used in early diagnosis of diseases as a new magnetic resonance contrast agent and a new fluorescent probe; it can also be used as a new drug vector that carries drugs highly efficiently and to conduct targeting treatment. Triple functions of the magnetic nano-materials coated with proteins are thereby achieved: (1) the highly uniform cage-structure of proteins enables the magnetic nano-particles to have highly uniform grain size and shape, high dispersity, water-solubility and biocompatibility; (2) the targeting ability of the ferritin shells coated outside is achieved; such targeting ability, different from that of conventional targeting magnetic nano-materials, is an inherent cells targeting ability without the need to link the material to targeting ligands or modify targeting ligands; (3) the magnetic nano-particles with high specific surface area and the core-shell structure of protein shells become a highly efficient drug delivery carrier. The invention is accomplished on the basis of the above concepts.

### SUMMARY OF THE INVENTION

The purpose of the present invention is to provide new applications of a cell-targeted magnetic nano-material in disease diagnosis and treatment.

In the first aspect of the present invention, the invention provides the use of a protein shell coated magnetic nano-particles or derivatives thereof in the preparation of imaging location diagnosis agents and treatment substances.

In another preferred embodiment, said imaging location diagnosis agent is selected from magnetic resonance contrast agent or molecular probe.

In another preferred embodiment, said magnetic resonance contrast agent or molecular probe comprises said protein shell coated magnetic nano-particles or derivatives thereof.

In another preferred embodiment, the composition of the core of said protein shell coated magnetic nano-particles or derivatives thereof is a compound comprising metal elements; said metal element is selected from gadolinium, manganese, iron, cobalt and/or nickel.

In another preferred embodiment, said protein shell of the protein shell coated magnetic nano-particles or derivatives thereof can bind with specificity to receptors expressed on the surface of tissues or cells; preferably, said protein shell is selected from ferritin, chaperone, DNA binding protein, magnetosome membrane protein of magnetotactic bacteria or viral protein shell having nano-cavity structure; preferably, said ferritin includes natural ferritin and genetic engineering recombinant ferritin, wherein the natural ferritin originates from eukaryotes or prokaryotes, and the genetic engineering recombinant ferritin includes recombinant ferritin composed solely of heavy (H) chain subunits, recombinant ferritin composed solely of light (L) chain subunits, recombinant ferritin composed of heavy chains and light chains in arbitrary proportion by self-assembly, and mutants or fusion proteins of such protein subunits.

In another preferred embodiment, said protein shell coated magnetic nano-particle or derivative thereof is prepared by following steps:
(a) using recombinant human ferritin as a template, cloning and constructing the full length cDNAs of H-subunit and L-subunit of human ferritin onto pET11b plasmids, respectively;
(b) adding isopropyl-β-D-thiogalactopyranoside to cells BL21(DE3)plysS separately transformed or co-transformed by recombinant plasmids comprising human ferritin H-subunits and L-subunits, to activate T7 promoter and induce expression;
(c) releasing the proteins by ultrasonication after expression;
(d) separating and purifying the proteins;
(e) adding metal salts that form the composition of the core and an oxidant to the solution of recombinant human ferritin to undergo reaction, controlling the pH at 7-11 and the temperature at 25-80°C, to form strongly magnetic nano-particles within the recombinant human ferritin, wherein the concentration of the salts that form the composition of the core is such that the ratio of the number of metal atoms added each time to the number of protein molecules is between 10-200, so that the number of metal atoms added in a single protein molecule is between 100-15000; the concentration of the oxidant is such that the ratio of the number of oxidant molecules to the number of added metal ions is 2:1 or 3:1; and the concentration of the proteins is greater than 0.25mg/mL; and
(f) obtaining the protein shell coated magnetic nano-particles or derivatives thereof after separation by size exclusion or ion-exchange chromatography and purification by centrifugation and molecular sieve or anion-exchange chromatography.

In another preferred embodiment, the pH is controlled at 8-9 in step (e).

In another preferred embodiment, the temperature is controlled at 35-70°C in step (e).

In another preferred embodiment, said metal salts that form the composition of the core are selected from ferrous salt, ferric salt, gadolinium salt, manganese salt, cobalt salt and/or nickel salt.

In another preferred embodiment, said metal element is selected from gadolinium, manganese, iron, cobalt and/or nickel.

In another preferred embodiment, said oxidant is selected from hydrogen peroxide, oxygen gas and substances that can produce hydrogen peroxide or oxygen gas through reaction.

In another preferred embodiment, the number of metal atoms added into a single protein molecule is between 140-10000.

In another preferred embodiment, the number of gadolinium atoms added into a single protein molecule is between 100-200, and/or the number of iron atoms added into a single protein molecule is between 500-10000.

In another preferred embodiment, said treatment substance is a substance for treating diseases that express ferritin receptors; preferably, said substance is linked to a protein shell that encapsulates magnetic nano-particles; more preferably, said substance is selected from chemotherapeutic drug, radioactive isotope, cytokine, nucleic acid and anti-cancer or anti-inflammation drug.

In another preferred embodiment, said disease that expresses ferritin receptors is tumor and/or inflammation; preferably, said tumor is selected from hepatocellular carcinoma, leukemic carcinoma, neuro glioma, pulmonary carcinoma, colonic carcinoma, pancreatic carcinoma or mammary carcinoma.

In the second aspect of the present invention, the invention provides the use of a protein shell coated magnetic nano-particles or derivatives thereof in the preparation of magnetic contrast agent and molecular probe for diagnosis of diseases that express ferritin receptors.

In another preferred embodiment, said disease that expresses ferritin receptors is tumor and/or inflammation; preferably, said tumor is selected from mammary carcinoma, hepatocellular carcinoma, pulmonary carcinoma, colonic carcinoma, pancreatic carcinoma, neuro glioma, leukemic carcinoma or prostatic carcinoma.

In the third aspect of the present invention, the invention provides a protein shell coated magnetic nano-particles or derivatives thereof; the composition of the core of said protein shell coated magnetic nano-particles or derivatives thereof is a compound comprising metal elements; said metal element is selected from gadolinium, manganese, iron, cobalt and/or nickel.

In another preferred embodiment, the composition of the core of said protein shell coated magnetic nano-particle or derivative thereof is a compound comprising iron.

In another preferred embodiment, the composition of the core of said protein shell coated magnetic nano-particle or derivative thereof is a compound comprising gadolinium.

In another preferred embodiment, the composition of the core of said protein shell coated magnetic nano-particle or derivative thereof is a compound comprising iron and manganese.

In another preferred embodiment, the composition of the core of said protein shell coated magnetic nano-particle or derivative thereof is a compound comprising iron and gadolinium.

In another preferred embodiment, said protein shell is selected from ferritin, chaperone, DNA binding protein, magnetosome membrane protein of magnetotactic bacteria or viral protein shell having nano-cavity structure; the protein shell of said protein shell coated magnetic nano-particles or derivatives thereof can bind with specificity to receptors expressed on the surface of tissues or cells; preferably, said ferritin comprises natural ferritin and genetic engineering recombinant ferritin, wherein the natural ferritin originates from eukaryotes or prokaryotes, and the genetic engineering recombinant ferritin comprises recombinant ferritin composed solely of heavy (H) chain subunits, recombinant ferritin composed solely of light (L) chain subunits, recombinant ferritin composed of heavy chains and light chains in arbitrary proportion by self-assembly, and mutants or fusion proteins of such protein subunits.

In the fourth aspect of the present invention, the invention provides a method for preparing a protein shell coated magnetic nano-particles or derivatives thereof, as provided hereinbefore, said method comprising following steps:
(a) using recombinant human ferritin as a template, cloning and constructing the full length cDNAs of H-subunit and L subunit of human ferritin onto pET11b plasmids, respectively;
(b) adding isopropyl-β-D-thiogalactopyranoside to cells BL21(DE3)plysS separately transformed or co-transformed by recombinant plasmids comprising human ferritin H-subunits and L-subunits, to activate T7 promoter and induce expression;
(c) releasing the proteins by ultrasonication after expression;
(d) separating and purifying the proteins;
(e) adding metal salts that form the composition of the core and an oxidant to the solution of recombinant human ferritin to undergo reaction, controlling the pH at 7-11 and the temperature at 25-80°C, to form strongly magnetic nano-particles within the recombinant human ferritin, wherein the concentration of the salts that form the composition of the core is such that the ratio of the number of metal atoms added each time to the number of protein molecules is between 10-200, so that the number of metal atoms added in a single protein molecule is between 100-15000; the concentration of the oxidant is such that the ratio of the number of oxidant molecules to the number of added metal ions is 2:1 or 3:1; and the concentration of the proteins is greater than 0.25mg/mL; and
(f) obtaining the protein shell coated magnetic nano-particles or derivatives thereof after separation by size exclusion or ion-exchange chromatography and purification by centrifugation and molecular sieve or anion-exchange chromatography.

In another preferred embodiment, the pH is controlled at 8-9 in step (e).

In another preferred embodiment, the temperature is controlled at 35-70°C in step (e).

In another preferred embodiment, said metal salts that form the composition of the core is selected from ferrous salt, ferric salt, gadolinium salt, manganese salt, cobalt salt and/or nickel salt.

In another preferred embodiment, said metal element is selected from gadolinium, manganese, iron, cobalt and/or nickel.

In another preferred embodiment, said oxidant is selected from hydrogen peroxide, oxygen gas and substances that can produce hydrogen peroxide or oxygen gas through reaction.

In another preferred embodiment, the number of metal atoms added into a single protein molecule is between 140-10000.

In another preferred embodiment, the atom number of gadolinium added into a single protein molecule is between 100-200, and/or the atom number of iron added into a single protein molecule is between 500-10000.

Thereby, the present invention provides new applications of a cell-targeted magnetic nano-material in disease diagnosis and treatment.

### BRIEF DESCRIPTION OF DRAWINGS

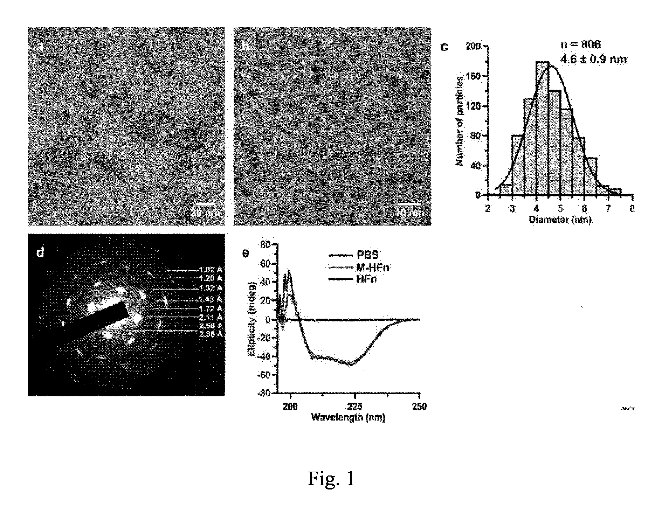

FIG. 1 shows the structure analysis of human H-subunit magnetoferritin comprising magnetite core (Fe₃O₄) prepared according to Embodiment 1, wherein a is the negative-stained electron microscope image; b is the electron microscope image of the cores; c is the histogram of granularity distribution; d is the selected area electron diffraction (SAED) image of the cores; e is the circular dichroism (CD) analysis of the protein configuration.

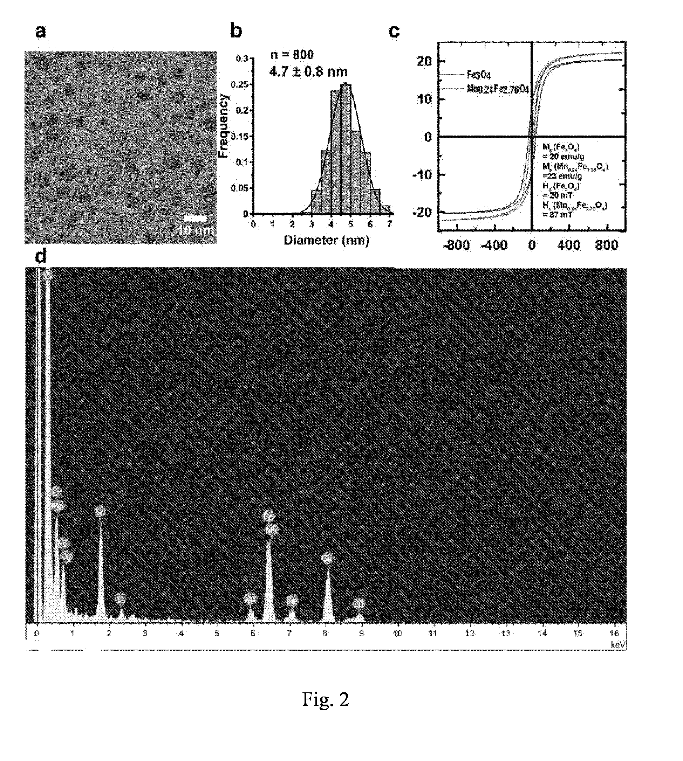

FIG. 2 illustrates the magnetic nano-material comprising ferritin shell coated manganese-iron oxide cores synthesized according to Embodiment 2, wherein FIG. 2a is the scanning electron microscope image of the cores of manganese-iron oxide; b is the histogram of the granularity distribution of the cores; c is the hysteresis loop of manganese-iron oxide (compared with the human H-subunit magnetoferritin with cores consisting of magnetite (Fe₃O₄)); d is the element analysis by energy dispersive spectroscopy of the cores of ferritin shell coated manganese-iron oxide.

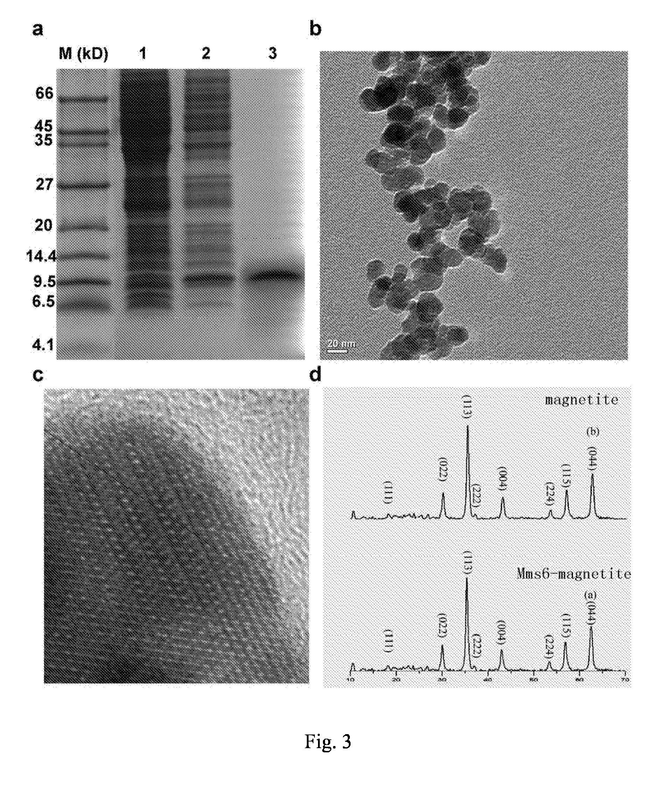

FIG. 3 illustrates the magnetic nano-material biomimetically synthesized by membrane protein Mms6 according to Embodiment 3, wherein FIG. 3a is the image of prokaryotic expression and purification of Mms6 protein labeled with His; b is the electron microscope image of magnetic nano-particles biomimetically synthesized by His-Mms6; c is the high-resolution electron microscope image (crystal lattice) of magnetic nano-particles biomimetically synthesized by His-Mms6; d is the X-ray crystallography (XRD) image of magnetic nano-particles biomimetically synthesized by His-Mms6.

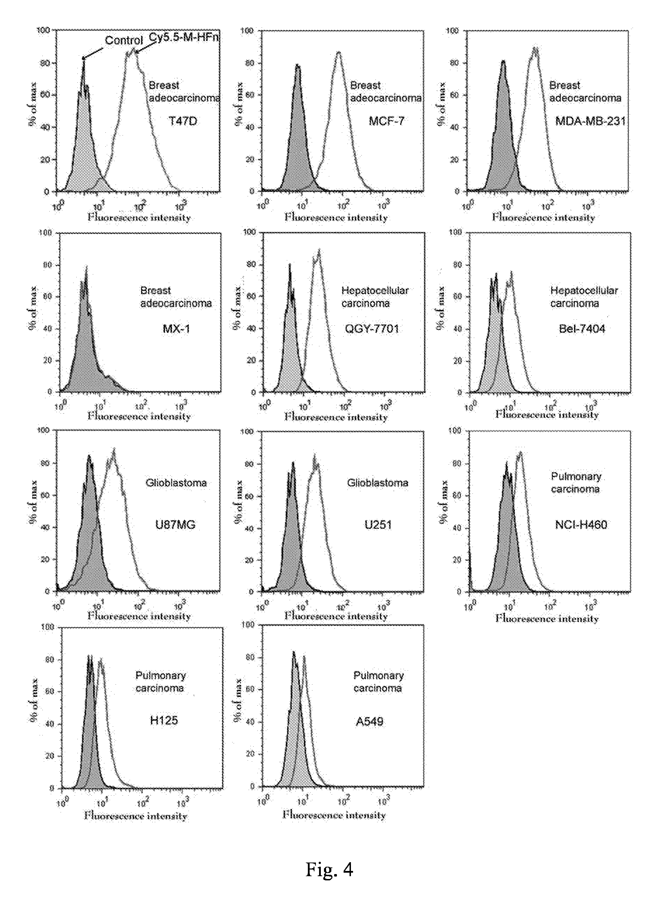

FIG. 4 illustrates the flow analysis of the specific binding of human H-subunit magnetoferritin to various highly expressing cells ferritin receptor.

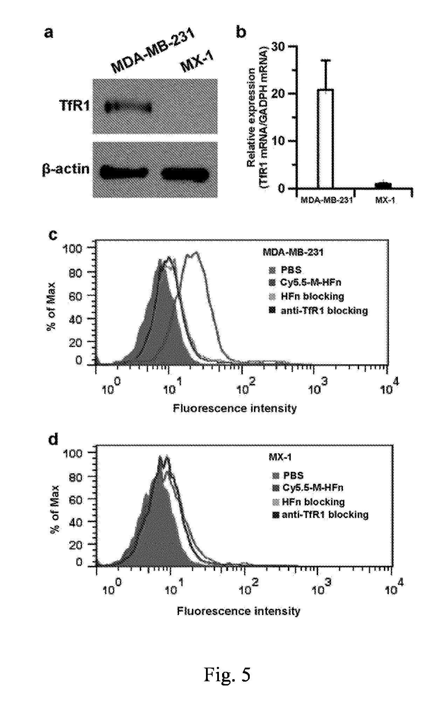

FIG. 5 illustrates the flow analysis of the specific binding and competitive inhibition between ferritin-receptor highly-expressed cells and human H-subunit magnetoferritin, wherein a-b illustrate that MDA-MB-231 cells highly expressing ferritin receptors and MX-1 cells expressing no ferritin receptors are detected and screened by using Western blotting and fluorescence quantitative PCR; c illustrates the flow analysis of the specific binding between human H-subunit magnetoferritin and the competitive inhibition between protein shells and anti-ferritin receptors; d illustrate the flow analysis of the binding between MX-1 cells and human H-subunit magnetoferritin.
FIG. 6 illustrates the measurement of transverse relaxation effect using human H-subunit magnetoferritin as a magnetic resonance contrast agent, wherein a is the T₂-weighted image of human H-subunit magnetoferritin with different iron concentrations; b is the fitting curve obtained from calculating the traverse relation ratio (R₂) with different iron concentrations.
FIG. 7 shows the MRI test of the ferritin receptors expressed in vitro using human H-subunit magnetoferritin as a magnetic resonance contrast agent, wherein a is the magnetic resonance image of the control group MDA-MB-231 cells (highly expressing ferritin receptors) without the material added; b is the MRI image of MDA-MB-231 cells incubated with the material for 5.5 h; c is the Prussian blue stained image of the control group MDA-MB-231 cells; e is the Prussian blue stained image of MDA-MB-231 cells incubated with the material for 5.5 h; e is the MRI image of MX-1 cells (expressing no ferritin receptors) without the material added; f is the MRI image of MX-1 cells incubated with the material for 5.5 h; g is the Prussian blue stained image of the control group MX-1 cells; h is the Prussian blue stained image of MX-1 cells incubated with the material for 5.5 h.

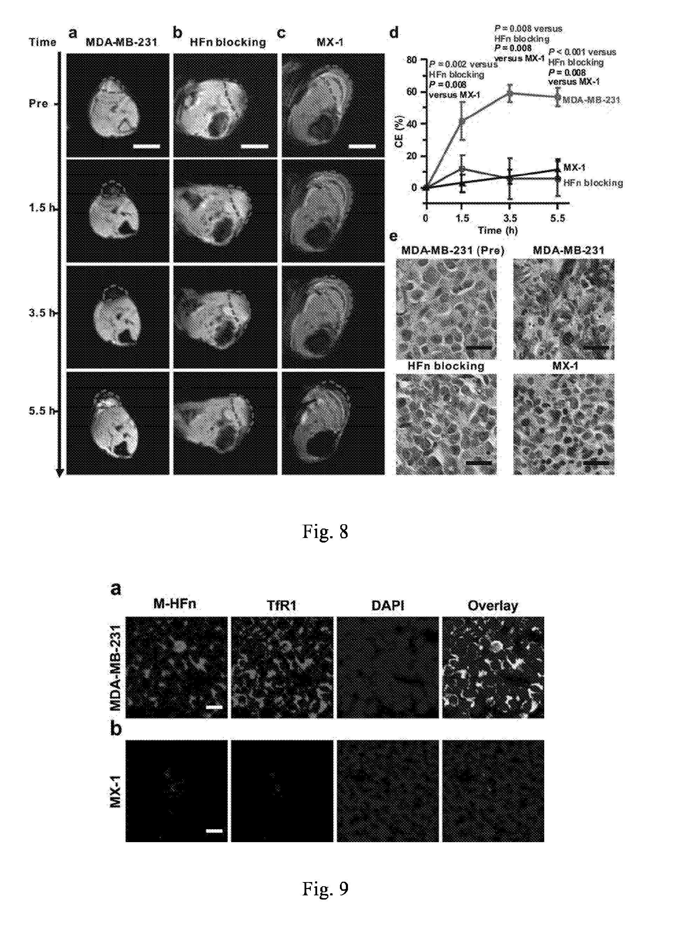

FIG. 8 illustrates the MRI analysis result that indicates that human H-subunit magnetoferritin can specifically target the tissues that express ferritin receptors in vivo, resulting in a significant change of the signal intensity of the MRI image; it is verified that the material specifically aggregates in tissues with histologic iron-staining result, whrerein FIGS. 8a-c are T₂* MRI images of a mouse bearing MDA-MB-231 tumor, a mouse bearing MDA-MB-231 tumor competitively inhibited with ferritin, and a mouse bearing MX-1 tumor that lowly expresses ferritin receptors; d illustrates the quantitative analysis results of T₂*-weighted MRI images of the tumors; e illustrates the iron-staining results of the tumor tissues of the mouse bearing MDA-MB-231, the mouse bearing MDA-MB-231 tumor competitively inhibited with ferritin, and the mouse bearing MX-1 tumor that lowly expresses ferritin receptors, wherein the scale is 20µm.
FIG. 9 illustrates the fluorescent tracking analysis of specific targeting mechanism of human H-subunit magnetoferritin, which is indicated to be through the binding of TfR1 highly expressed by tumor cells, wherein a shows the fluorescence co-localization images of ferritin-receptor highly-expressed MDA-MB-231 tumor; b shows the fluorescence co-localization images of MX-1 tumor that lowly expresses ferritin receptors, wherein the scale is 50µm.

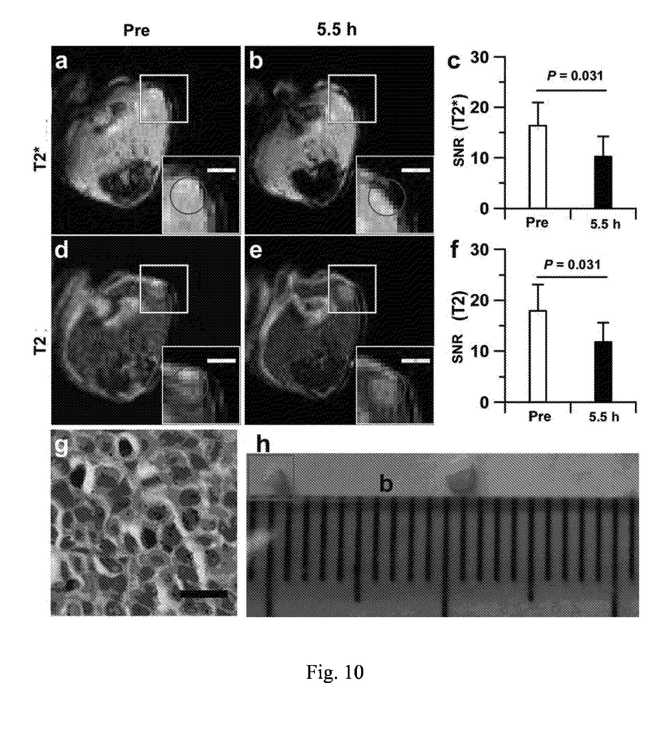

FIG. 10 illustrates the use of human H-subunit magnetoferritin in the early diagnosis of an MDA-MB-231 human mammary carcinoma with the size about 1 mm as a tumor-targeted magnetic resonance contrast agent, wherein
FIGS. 10a, b are the T₂*-weighted images of nude mice bearing MDA-MB-231 minute carcinoma lesion; FIG. 8c illustrates quantitative analysis result and statistical analysis result of the T₂*-weighted images; FIGS. 8d-f illustrate the quantitative analysis results of SNR in T₂-weighted magnetic image; FIG. 8g shows the iron-staining result of the carcinoma lesion paraffin slice stained with DAB-enhanced Prussian blue, wherein the scale is 20µm; FIG. 8h is the image of MDA-MB-231 minute carcinoma lesion after being dissected out.

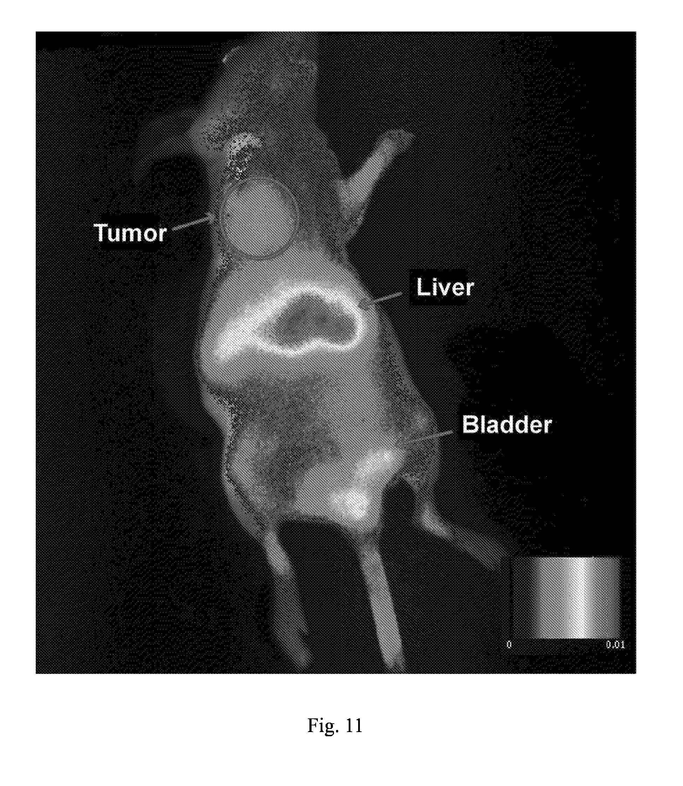

FIG. 11 illustrates the use of Cy 5.5 linked human H-subunit magnetoferritin in the early diagnosis of MDA-MB-231 human mammary carcinoma with the size about 3mm as a fluorescence molecular probe.

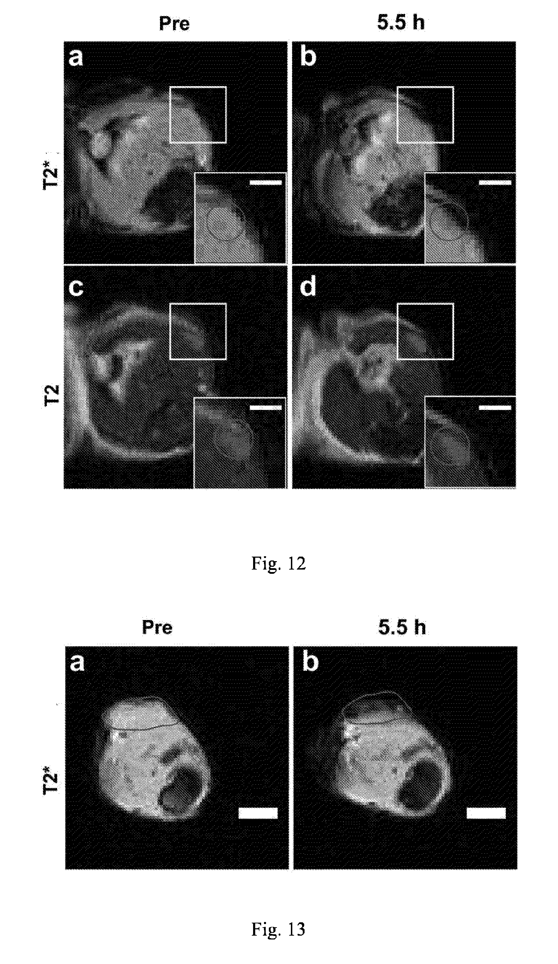

FIG. 12 illustrates the use of human H-subunit magnetoferritin in the early diagnosis of minute hepatocellular carcinoma, wherein the scale is 1 mm and the image in the lower right corner is a close-up image of the tumor.
FIG. 13 illustrates the use of human H-subunit magnetoferritin in the early diagnosis of minute pulmonary carcinoma, wherein the scale is 2mm.

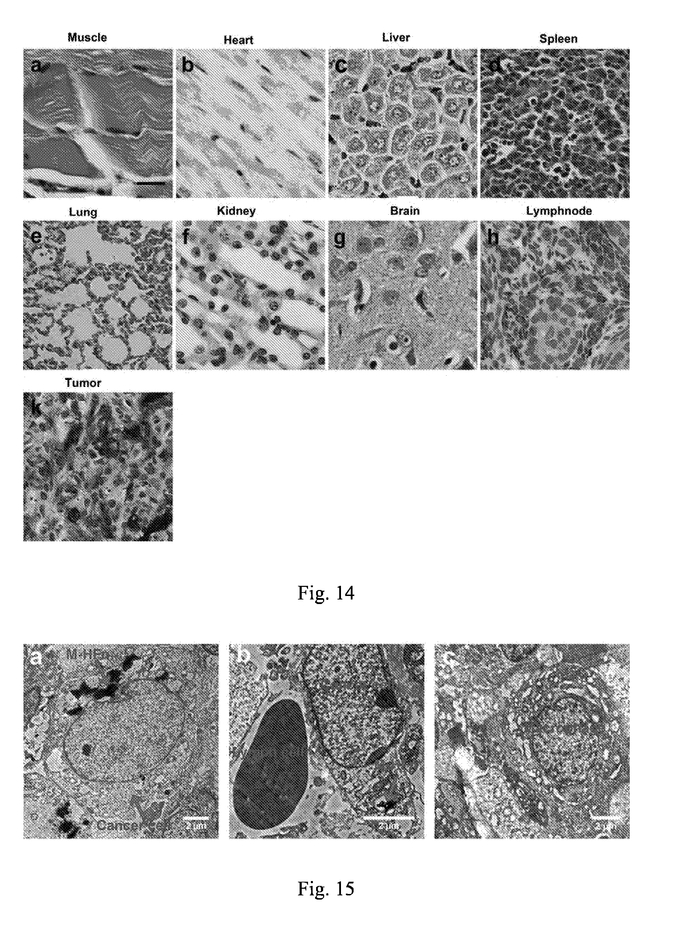

FIG. 14 illustrates the distribution of human H-subunit magneto ferritin in the tissues and organs of the nude mouse, wherein the scale is 20µm.
FIG. 15 illustrates the TEM result of the ultra-thin slices of tumor tissues, which indicates that the drug-linked material aggregates specifically in the tumor, and is a magnetic nano-material internalized by tumor cells, wherein FIG. 15a is an electron microscope image of the tumor cells in tumor tissues; b is an electron microscope image of tumor cells and lymph cells; c is an electron microscope image macrophages.

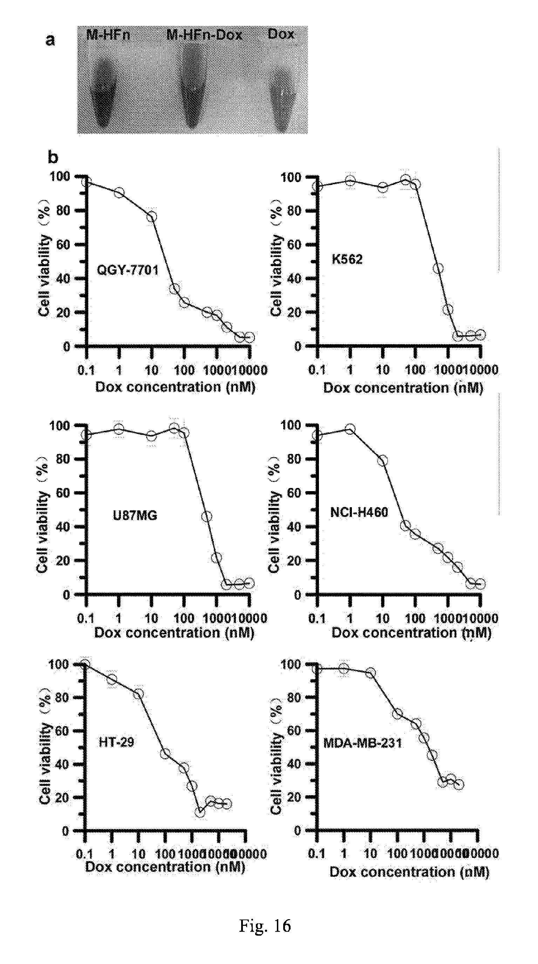

FIG. 16 illustrates the cytotoxicity experiment of human H-subunit magnetoferritin carrying the chemotherapeutic drug doxorubicin hydrochloride to various tumor cells (tested by MTT method), wherein FIG. 16a shows the color changes of the material after being linked to drugs; b illustrates the influence, tested by MTT, of the doxorubicin-linked material on the survival rate of hepatocellular, leukemic, glioma, pulmonary, colonic and mammary carcinoma cells.

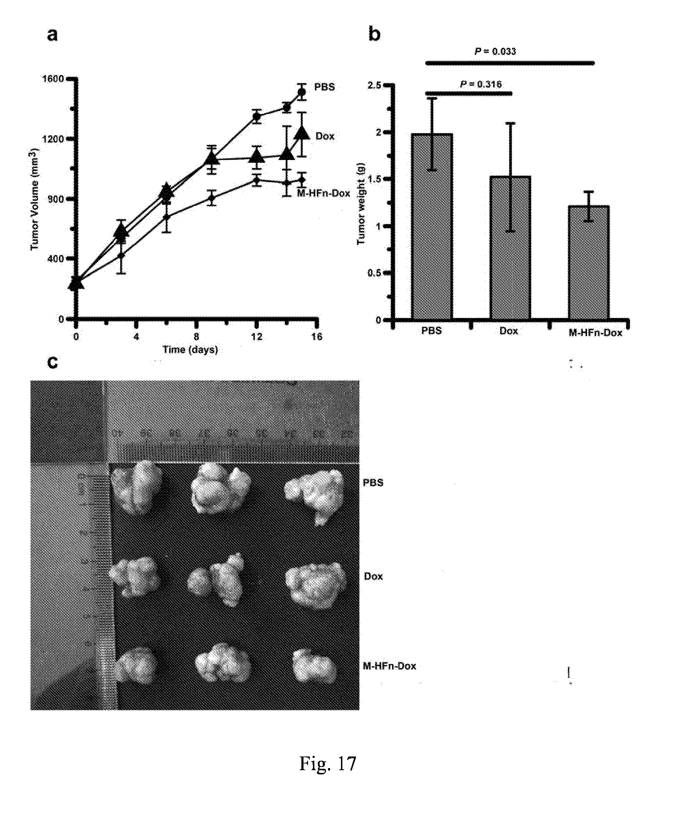

FIG. 17 illustrates the preliminary results of the treatment experiment on pulmonary carcinoma in vivo using doxorubicin-linked human H-subunit magnetoferritin, wherein FIG. 17a is a graph of the volumes of tumors versus days after being injected with the drugs; b illustrates the real weights of the tumors dissected out from different groups; c is the physical image of the tumors dissected out from different groups.

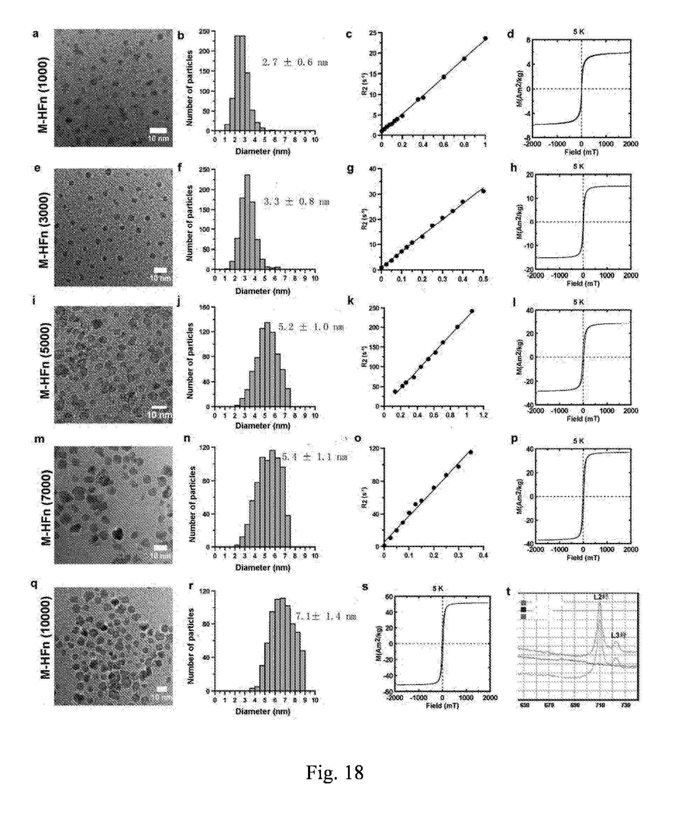

FIGS. 18a, 18b, 18c and 18d are the transmission electron microscope (TEM) image, the histogram of grain size distribution, the transverse relaxation rate (r₂) diagram and the low temperature (5K) magnetic hysteresis loop, respectively, of the magnetoferritin synthesized in the reaction in which an average of 1000 iron atoms are added into a single protein molecule. FIGS. 18e, 18f, 18g and 18h are the transmission electron microscope (TEM) image, the histogram of grain size distribution, the transverse relaxation rate (r₂) diagram and the low temperature (5K) magnetic hysteresis loop, respectively, of the magnetoferritin synthesized in the reaction in which an average of 3000 iron atoms are added into a single protein molecule. FIGS. 18i, 18j, 18k and 18l are the transmission electron microscope (TEM) image, the histogram of grain size distribution, the transverse relaxation rate (r₂) diagram and the low temperature (5K) magnetic hysteresis loop, respectively, of the magnetoferritin synthesized in the reaction in which an average of 5000 iron atoms are added into a single protein molecule. FIGS. 18m, 18n, 18o and 18p are the transmission electron microscope (TEM) image, the histogram of grain size distribution, the transverse relaxation rate (r₂) diagram and the low temperature (5K) magnetic hysteresis loop, respectively, of the magnetoferritin synthesized in the reaction in which an average of 7000 iron atoms are added into a single protein molecule. FIGS. 18q, 18r, and 18s are the transmission electron microscope (TEM) image, the histogram of grain size distribution and the low temperature (5K) magnetic hysteresis loop, respectively, of the magnetoferritin synthesized in the reaction in which an average of 10000 iron atoms are added into a single protein molecule FIG. 18t is the electron energy loss spectroscopy (EELS) of the magnetoferritin synthesized by adding an average of 5000 iron atoms into a single protein molecule (average grain size 5.2nm).

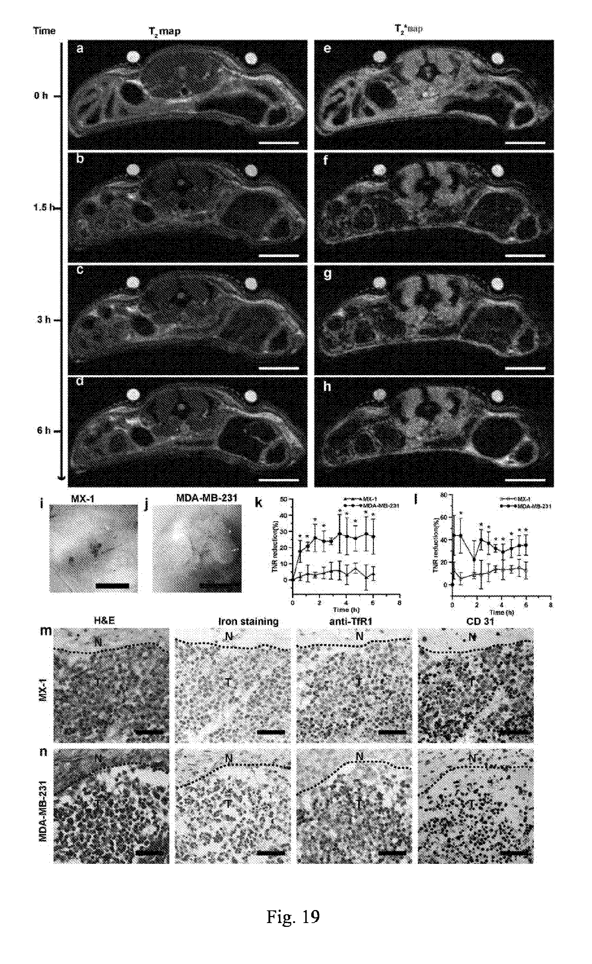

FIGS. 19a-d are the T₂-weighted MRI images of a nude mouse model with a mammary carcinoma of about 3 mm. FIGS. 19e-h are the T2*-weighted MRI images of a nude mouse with a tumor of about 3 mm (scale: 5mm; the tumors are marked with red circles). FIGS. 19i and 19j are the in-situ stereoscopic microscope images of the tumor (scale: 3 mm). FIGS. 19k and 19l illustrate the quantitative analysis of the T₂-weighted images and the T₂*-weighted images (statistics of 4 models) respectively. FIGS. 19m and 19n are the immunohistochemistry images of the tumors after magnetic resonance scanning (N stands for normal tissues, and T stands for tumor tissues; scale: 50µm).

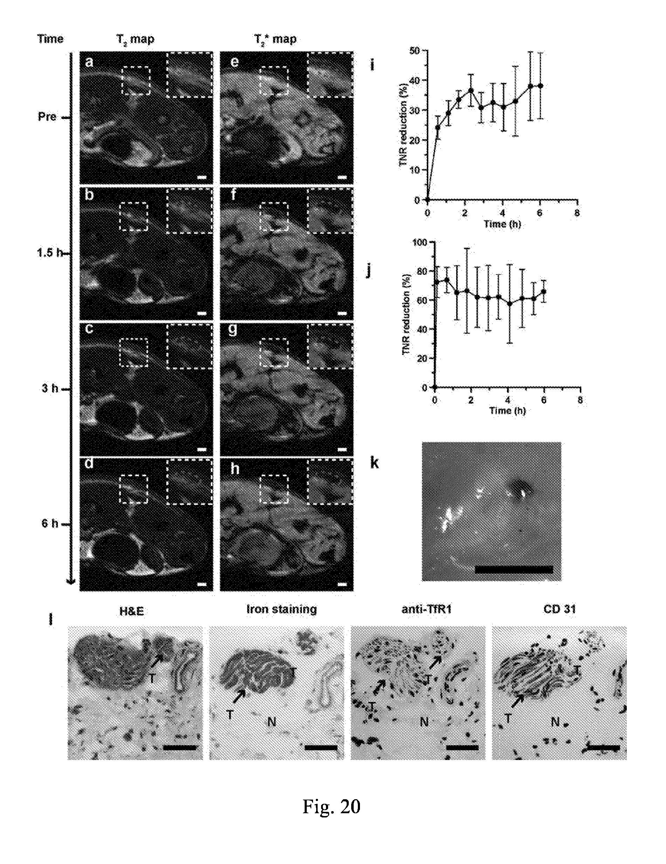

FIGS. 20a-d are the T₂-weighted MRI images of a nude mouse model with a mammary carcinoma of about 1 mm. FIGS. 20e-h are the T₂*-weighted MRI images of a nude mouse with a tumor of about 1 mm (scale: 1 mm; the tumors are marked with red circles). FIGS. 20i and 20j show the quantitative analysis of the T₂-weighted images and the T₂*-weighted images (statistics of 4 models). FIG. 20k is the in-situ stereoscopic microscope image of a subcutaneous transplantation mammary carcinoma (scale: 1 mm). FIG. 20l shows the immunohistochemistry of the tumor after magnetic resonance scanning (N stands for normal tissues, and T stands for tumor tissues. Scale: 50µm).

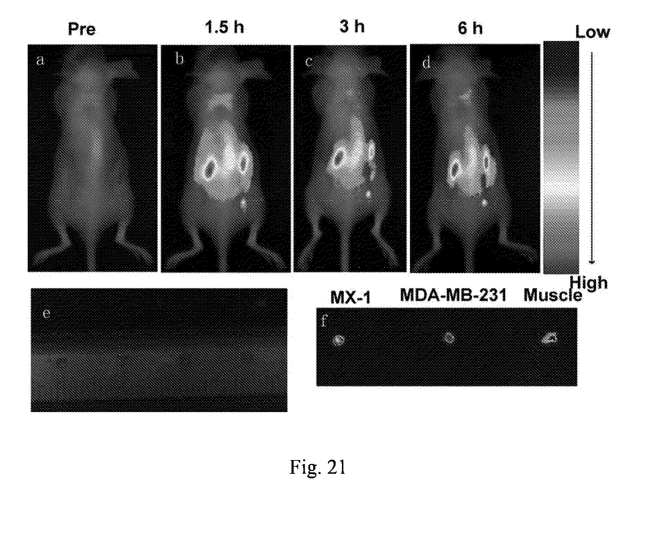

FIGS. 21a-21d are the fluorescence imaging images of nude mice models with mammary carcinoma of about 3 mm prior to the injection of Cy5.5 ferritin and 1.5h, 3h, and 6h after injection (the tumors are marked with red circles). FIG. 21e and FIG 21f are the white light images and the fluorescence images, respectively, of the minute MX-1 tumor, MDA-MB-231 tumor and surrounding normal muscle tissues dissected out.

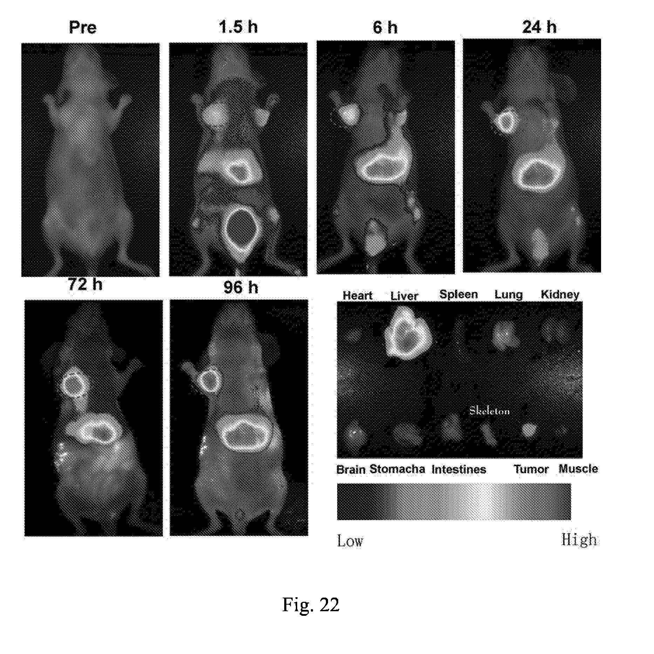

FIG. 22 shows the near infrared fluorescence in vivo imaging images of the nude mice models with tumors of about 3 mm (the tumors are marked with red circles) injected with Cy5.5 ferritin, and the fluorescence imaging images of individual tissue or organ (heart, liver, spleen, lung, kidney, brain, stomach, intestine, bone, tumor and muscle) dissected out after 96h.

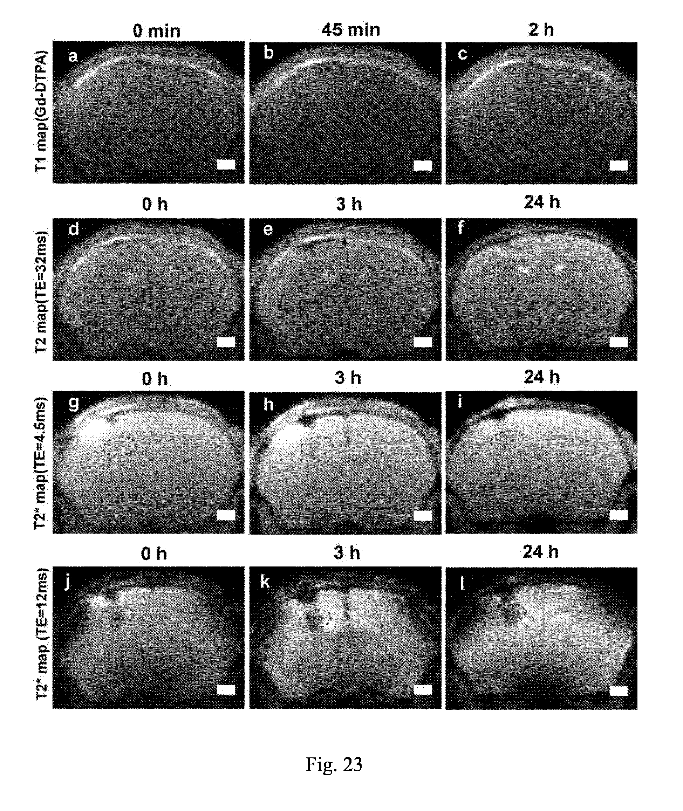

FIGS. 23a-23c are the magnetic resonance scanning images of the nude mice models with in-situ glioma of about 1-2 mm prior to and after Gd-DTPA injection. FIGS. 23d-f are the T₂-weighted magnetic resonance imaging images (echo time 32 ms) prior to and after magnetoferritin injection. FIGS. 23g-23i are the T₂*-weighted magnetic resonance imaging images (echo time 4.5 ms). FIGS. 23j-23l are the second T₂*-weighted magnetic resonance imaging images (echo time 12 ms). (Scale: 2 mm; the tumors are marked with red circles.)

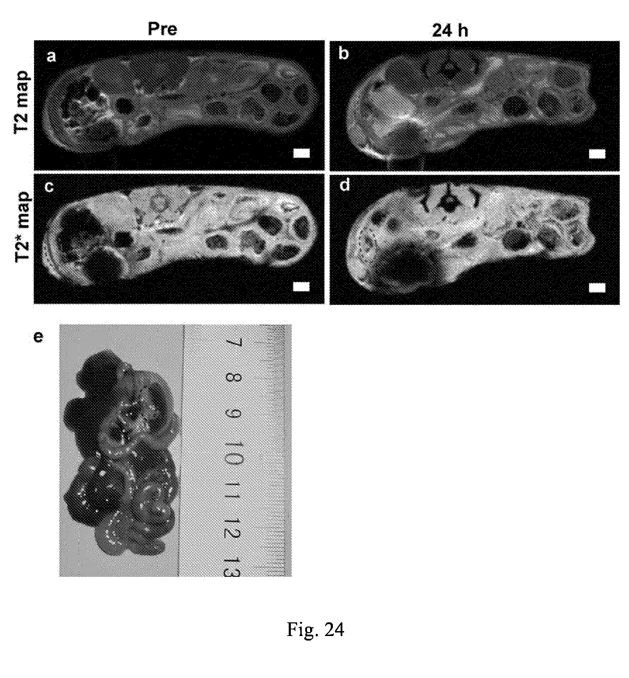

FIGS. 24a-24d are the T₂-weighted and T₂*-weighted magnetic resonance imaging images of the in-situ pancreatic carcinoma implanted nude mouse model prior to and after magnetoferritin injection, respectively (scale: 2 mm; the tumors are marked with red circles). FIG. 24e is the in-situ observation image of the abdominal cavity organs dissected out from the nude mice in supine position after magnetic resonance scanning (the tumors in pancreas are marked with blue circles).

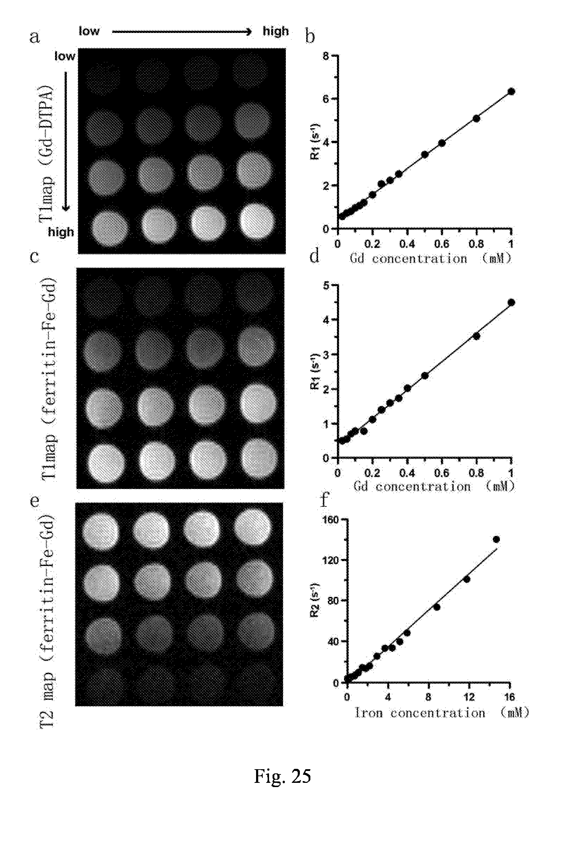

FIG. 25a is the T₁-weighted magnetic resonance imaging images (echo time TE = 8.5 ms, reversal time TR = 300 ms) of commercialized Gd-DTPA (magnevist, produced by Bayer Corp.) with various gadolinium concentrations (0-1 mM). FIG. 25b illustrates the linear relation between the reciprocals of the measured longitudinal relaxation time (R₁ = 1/T₁) of Gd-DTPA with various gadolinium concentrations. FIG. 25c shows the T₁-weighted imaging images (TE = 8.5 ms; TR = 300 ms) of ferritin-gadolinium-iron composite various gadolinium concentrations (0-1 mM). FIG. 25d illustrates the linear relation between the reciprocals of the measured longitudinal relaxation time (R₁ = 1/T₁) of ferritin-gadolinium-iron composite with various gadolinium concentrations. FIG. 25e is the T₂-weighted imaging images for various iron concentrations. FIG. 25f illustrates the linear relation between the reciprocals of the measured transverse relaxation time (R₂ = 1/T₂) of ferritin-gadolinium-iron composite with various iron concentrations.

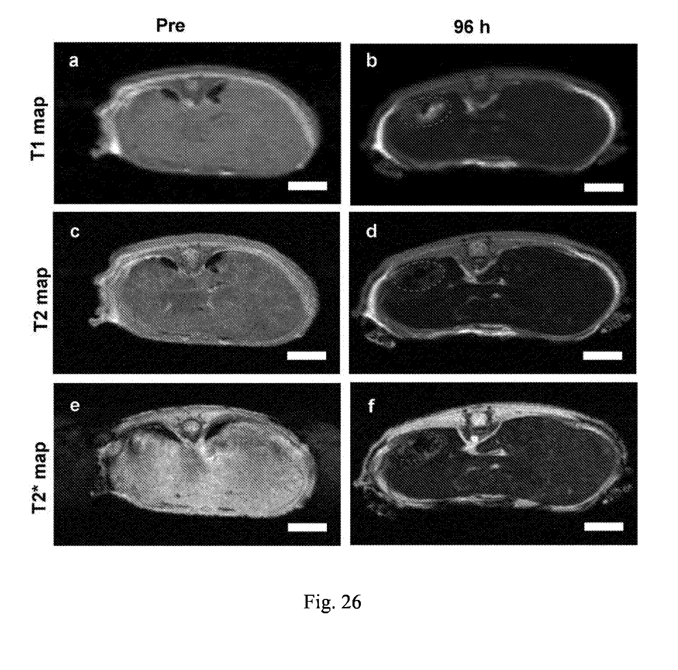

FIGS. 26a-26b are the T₁-weighted magnetic resonance imaging images of the in-situ hepatocellular carcinoma nude mouse transplantation model prior to and after the injection of ferritin-gadolinium-iron dual-mode magnetic resonance contrast agent, respectively. Fig. 26c-26d and Fig. 26e-26f are the T₂-weighted and T₂*-weighted magnetic resonance imaging images of the in-situ hepatocellular carcinoma nude mouse transplantation model prior to and after the injection of ferritin-gadolinium-iron dual-mode magnetic resonance contrast agent, respectively (scale: 5 mm; the tumors are marked with red circles).

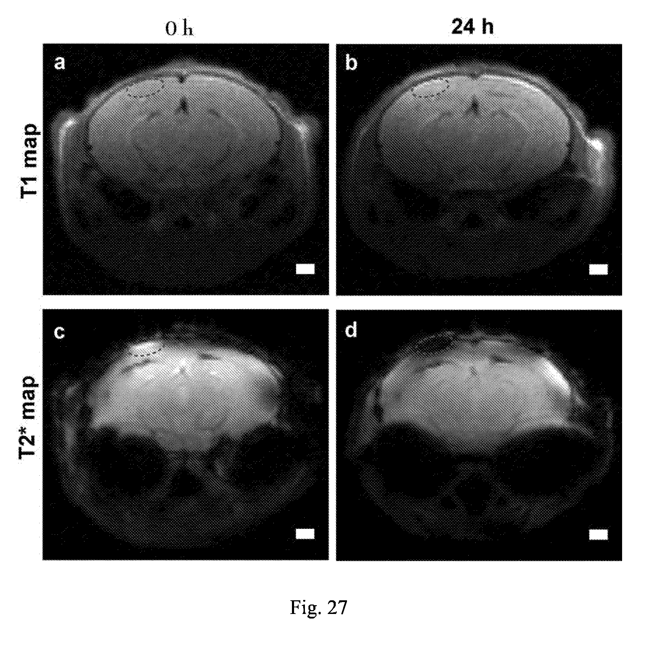

FIGS. 27a-27b are the T₁-weighted magnetic resonance imaging images of the in-situ glioma nude mouse transplantation model prior to and after the injection of ferritin-gadolinium-iron dual-mode magnetic resonance contrast agent, respectively. FIGS. 27c-27d are the T₂*-weighted magnetic resonance imaging images of the in-situ glioma nude mouse transplantation model prior to and after the injection of ferritin-gadolinium-iron dual-mode magnetic resonance contrast agent (scale: 2 mm; the tumors are marked with red circles).

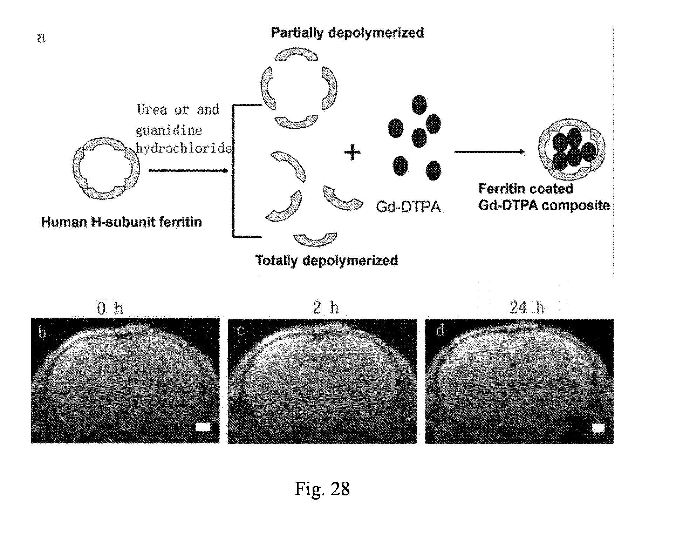

FIG. 28a is the schematic chart for the process of human H-subunit ferritin encapsulating Gd-DTPA. FIGS. 28b-28d are the T₁-weighted magnetic resonance imaging images of the in-situ glioma nude mice transplantation models prior to and after the injection of human H-subunit ferritin coated Gd-DTPA composite (scale: 2 mm; the tumors are marked with red circles).

### DETAILED DESCRIPTION

After extensive and in-depth research, on the basis of Chinese invention patent applications 200910244505.1 and 201010034208.7, the inventors use human source magnetoferritin biomimetically synthesized using genetic engineering recombinant in diagnosis and treatment of diseases highly expressing ferritin receptors, and the human source magnetoferritin is synthesized biomimetically by human H-subunit ferritin from genetic engineering recombinant expression. The present invention is accomplished on this basis.

Diagnosis of diseases includes in vivo imaging, in vitro tissue and cell diagnosis based on magnetic nano-material or derivatives thereof. Treatment of diseases includes passive targeting treatment and active targeting treatment based on magnetic nano-material or derivatives thereof.

As used herein, "core component" refers to "strongly magnetic nano-particles" or "magnetic nano-material".

As used herein, "magnetic nano-material" is the protein shell coated magnetic nano-material prepared using the method of the present invention and the method provided by Chinese invention patent application 200910244505.1. Said magnetic nano-material is cell targeted; said cell targeting is inherent so that the material can directly bind with specificity to the cells having high expression on ferritin receptors without surface coating, chemical modification or genetic engineering modification to link to targeting ligands (such as antibody, polypeptide, targeting small molecule etc.).

Ferritin and fusion ferritin provide natural targeting ligands for the material. Said ferritin includes natural ferritin and genetic engineering recombinant ferritin, wherein the natural ferritin originates from eukaryotes or prokaryotes, and the genetic engineering recombinant ferritin includes recombinant ferritin composed solely of heavy (H) chain subunits, recombinant ferritin composed solely of light (L) chain subunits, recombinant ferritin composed of heavy chains and light chains in arbitrary proportion by self-assembly, mutants or fusion proteins of such protein subunits, and the cell targeting ability of other protein biomimetically synthesized magnetic materials or non-magnetic materials.

Said cell targeting comprises in vivo and in vitro cell targeting, enabling the material to bind with specificity to cells highly expressing ferritin receptors and to enter into tumor cells by endocytosis; the in vivo and in vitro cell targeting is realized by the magnetic nano-material of the present invention binding with specificity to ferritin receptors having high expression on cell surface.

The magnetic nano-material or derivatives thereof provided by the present invention has a complete human H-subunit ferritin shell and a core comprising magnetite and/or maghemite, manganese iron oxide, gadolinium-iron composite and Gd-DTPA. The ferritin shell has an outer diameter of 12-15 nm, a highly uniform size and a spherical shape. The grain size of the core can be altered by controlling the number of iron atoms added into a single protein molecule, thus enabling the synthesis of magnetoferritin with various gain sizes. Said material has a grain size distribution within a narrow range of 2-8 nm, an approximately spherical shape, good monodisperstiy and water solubility. The transverse relaxation time (r₂) of said material can be altered by synthesizing magnetoferritin with various granularities, and r₂ can be controlled within the range of about 20-350 mM⁻¹s⁻¹.

In the present invention, by further altering the core, a ferritin shell coated manganese iron oxide magnetic nano-material is synthesized. Said material has a grain size (4.7 ± 0.8 nm) similar to that of the original magnetoferritin having a magnetite core, but the saturation magnetization is enhanced and the coercivity is lowered.

In the present invention, by further altering the core, a ferritin-gadolinium-iron dual-mode magnetic resonance contrast agent magnetic nano-material is synthesized. Said material not only has the function of T₁ magnetic resonance contrast agent so as to be used in magnetic resonance T₁-weighted imaging, brightening targeted area, but also has the function of T₂ magnetic resonance contrast agent so as to be used in magnetic resonance T₂-weighted imaging, darkening targeted area.

In the present invention, by further using the self-assembly function of the ferritin shell, Gd-DTPA is encapsulated into the cavity of the ferritin to form a ferritin coated Gd-DTPA composite, acting as a T₁ magnetic resonance contrast agent.

In the present invention, by using the membrane protein Mms6 of magnetotactic bacteria having mineralization function for prokaryotic expression and purification, and using the purified Mms6 protein, a magnetite material having an approximately spherical shape and a grain size of about 20 nm is biomimetically synthesized.

In the present invention, by using flow cytometry, it is verified that mammary carcinoma, hepatocellular carcinoma, glioma and pulmonary carcinoma cells highly expressing ferritin receptors can bind to massive human H-subunit magnetoferritin, indicating that said material can bind with specificity to a broad spectrum of cells highly expressing ferritin receptors.

Through experiments on the specific binding between cells and the material and on competitive inhibition, it is verified that the binding between the material and cells is specific. Through competitive inhibition experiment of anti-transferrin receptor 1 (TfR1) antibody, it is verified that the ferritin receptor to which human H-subunit magnetoferritin specifically binds is TfR1 having high expression on the surface of tumor cells. Through cell magnetic resonance imaging experiment, it is verified that by using human H-subunit magnetoferritin as contrast agent, TfR1-positive MDA-MB-231 cells with a high expression and a concentration of 10⁶ cells/mL can be clearly detected.

By using nude mouse tumor transplantation models, magnetic resonance imaging (MRI) and fluorescence tracing, it is in vivo verified that human H-subunit magnetoferritin can aggregate to tumor tissues in massive amount. By using fluorescence co-localization, it is verified that the aggregation mechanism of the in vivo tumor tissues is a specific targeting toward TfR1 having high expression on the surface of tumor cells. The aggregation of human H-subunit magnetoferritin to the tumor can result in an evident change in the signal intensity of MRI image of the tumor area.

In the present invention, by using human H-subunit magnetoferritin as tumor targeted magnetic resonance contrast agent, early diagnosis of tumor with a size of about 1 mm is realized on nude mouse human mammary carcinoma, hepatocellular carcinoma and lung carcinoma models.

In the present invention, by using human H-subunit magnetoferritin, ferritin-gadolinium-iron composite and ferritin coated Gd-DTPA composite as tumor targeted magnetic resonance contrast agent, early diagnosis of hepatocellular carcinoma, glioma and pancreatic carcinoma is realized on nude mouse in-situ hepatocellular carcinoma, in-situ glioma and in-situ pancreatic carcinoma allo-transplantation models. Glioma and pancreatic carcinoma with a size of 1 mm can form clear images, having sharp tissue contrast against surrounding normal tissues.

In the present invention, human H-subunit magnetoferritin linked with fluorescent molecules is used as fluorescent molecular probe in near infrared fluorescence imaging, enabling the detection of human mammary carcinoma with a size of 3 mm on nude mouse model.

In the present invention, human H-subunit ferritin linked with fluorescent molecules is used as fluorescent molecular probe in near infrared fluorescence imaging, enabling the detection of human mammary carcinoma with a size of 1 mm on nude mouse model.

In the direct observation of ultrathin slices using transmission electron microscope, intravenously injected human H-subunit magnetoferritin can enter into tumor cells highly expressing TfR1, thus making a tumor cell internalizing magnetic nano-material.

In the present invention, human H-subunit magnetoferritin is linked with chemotherapeutic drug adriamycin hydrochloride. Cytotoxicity MTT experiment indicates that the material linked with adriamycin hydrochloride has a broad-spectrum inhibitive effect on tumor cells such as hepatocellular carcinoma cells, leukemic carcinoma cells, glioma cells, lung carcinoma cells, colon carcinoma cells and mammary carcinoma cells etc.

In the present invention, the material linked with chemotherapeutic drug is used in the in vivo treatment of a lung carcinoma nude mouse transplantation model. The growth of tumor can be significantly suppressed after intravenous injection.

Specifically, the present invention relates to a tumor targeted magnetic nano-material and biomedical uses thereof, featuring a new biomimetically synthesized material having a magnetic nano-core coated with a protein shell. The new biomimetically synthesized material is inherently tumor targeted and can be used as a tumor targeted magnetic resonance contrast agent, a molecular probe and a vector of medicine for early diagnosis and treatment of tumors.

Wherein said protein shell coated magnetic nano-particles are preferably prepared by a specific method. Said specific method is disclosed in the Chinese patent application 200910244505.1, said disclosure incorporated into the present application.

Wherein said protein shell is selected from ferritin, chaperone, DNA binding protein, magnetosome membrane protein of magnetotactic bacteria or viral protein shell having a nano-cavity structure.

Wherein said ferritin comprises natural ferritin and genetic engineering recombinant ferritin, wherein the natural ferritin originates from eukaryotes or prokaryotes, and the genetic engineering recombinant ferritin comprises recombinant ferritin composed solely of heavy (H) chain subunits, recombinant ferritin composed solely of light (L) chain subunits, recombinant human ferritin composed of heavy chains and light chains in arbitrary proportion by self-assembly, and mutants or fusion proteins of such protein subunits. Preferably, said ferritin is genetic engineering recombinant human ferritin composed solely of heavy chain subunits.

Wherein the amino acid sequence of said human H-subunit ferritin is an amino acid sequence comprising

MTTASTSQVRQNYHQDSEAAINRQINLELYASYVYLSMSYYFDRDDVALK
NFAKYFLHQSHEEREHAEKLMKLQNQRGGRIFLQDIKKPDCDDWESGLNA
MECALHLEKNVNQSLLELHKLATDKNDPHLCDFIETHYLNEQVKAIKELG
DHVTNLRKMGAPESGLAEYLFDKHTLGDSDNES.

Wherein said ferritin has a feature that 12 or 24 heavy chain subunits and light chain subunits self-assemble in arbitrary proportion to form a cage structure.

Wherein the composition of said magnetic nano-core comprises but is not limited to magnetic nano-materials comprising gadolinium, manganese, iron, cobalt and/or nickel. Preferably, the composition of the magnetic nano-core is magnetite and/or maghemite, manganese iron oxide, gadolinium-iron composite or Gd-DTPA.

Wherein the inherent cell targeting comprises the inherent cell targeting of ferritin shell and the inherent cell targeting of all magnetic and non-magnetic materials synthesized based on ferritin shell.

Wherein the inherent cell targeting refers to that the ferritin shell can bind with specific targeting to cells highly expressing ferritin receptors.

Wherein the ferritin receptor comprises TfR1, H-subunit ferritin receptor, L-subunit ferritin receptor and lactoferritin receptor thereof.

Wherein the inherent cell targeting enables specific binding to cells highly expressing ferritin receptors and endocytosis into the cells.

Wherein the biomedical uses comprise agents for tumor diagnosis and treatment developed based on the cell targeting of protein shell and materials synthesized with protein shell.

Wherein the tumor diagnosis agent comprises in vitro and in vivo tumor diagnosis agents.

Wherein the in vitro tumor diagnosis agent comprises tumor diagnosis agents used on tissues, cells, blood, urine, feces and several other secretions.

Wherein the in vivo tumor diagnosis agent relates to imaging location diagnosis for human tumors by targeting ferritin receptors, and comprises all MRI contrast agents, fluorescent molecular probes, isotope probes etc. synthesized based on protein shell.

Wherein the tumor treatment agent relates to a pharmaceutical carrier linked with protein shell coated magnetic nano-material, and relaters to chemotherapeutic drugs, radioactive isotopes, cytokines, nucleic acids and anti-cancer or anti-inflammation drugs based on protein shell coating.

As used herein, a pharmaceutical carrier may comprise any and all physiologically suitable solvents, dispersion media, coats, iso-osmotic and absorption retarding agents etc. Preferably, said vector is suitable for intravenous, muscular, subcutaneous, parenteral, spinal or epidermal use.

The innovative features of the present invention may include: (1) a magnetic ferritin having primitive protein configuration is biomimetically synthesized using human H-subunit ferritin, without destroying the structure and functions of the protein; (2) by using recombinant magnetotactic bacteria membrane protein Mms6, the synthesis process is improved so that a magnetite having a crystal form similar to the cubic octahedral crystal form of magnetotactic bacteria magnetosome and a uniform grain size can be synthesized at room temperature and atmospheric pressure; (3) the biggest differences between the magnetoferritin of the present invention and the prior magnetoferritins are revealed: ① the purity of the mineral phase is ensured so that all that is formed within the protein shell is ferromagnetic magnetite with a high magnetic susceptibility, and the magnetic hysteresis loop reaches saturation at a very weak magnetic field; ② it is discovered for the first time and verified that the magnetoferritin of the present invention has an inherent in vivo and in vitro cell targeting ability without any modification, and it is verified that the cell targeting ability of the material results from that the protein shell thereof can bind with specificity to ferritin receptors TfR1 having high expression on cell surface; (4) compared with magnetic nano-material prepared by conventional chemical synthesis and modified with targeting ligands, the inherent cell targeting ability does not require complex surface coating, targeting molecule chemical modification or targeting peptide genetic engineering modification; (5) by using protein shell coated magnetic nano-material as cell targeted magnetic resonance contrast agent, magnetic resonance imaging diagnosis of tumor with a size of about 1 mm is realized on nude mouse models, which is the best internationally reported early tumor diagnosis result at the present time; (6) it is verified that the material can enter into tumor cells, laying a foundation for in-cell tumor treatment; and by modifying the material with tumor chemotherapeutic drugs, it is found that the material has a broad spectrum inhibition and cytotoxicity on tumor cells on in vitro cell models and evident therapeutic effect on in vivo lung cancer.

The above-mentioned features of the present invention, or the features of the embodiments, can be combined in any manner. All the features disclosed in the present specification can used with any form of composition. Every feature disclosed in the specification can be substitute for any substitutive feature that can provide the same, equivalent or similar purpose. Therefore, unless otherwise specified, all the features disclosed are merely generic examples of equivalent or similar features.

The main advantages of the present invention reside in:
1. this type of material is a magnetic nano-material biomimetically synthesized based on biomineralization of proteins, having unique material science advantages such as uniform grain size and shape, complete protein shell, monodispersity, water solubility and high biological compatibility etc.;
2. the biggest difference between this type of material and prior biomimeticaly synthesized material and chemically synthesized material lies in that this material, unlike regular magnetic nano-material, does not require complex surface coating and targeting molecule chemical modification or genetic engineering modification to have a cell active targeting ability which is thereby inherent;
3. this type of material can in vivo and in vitro specifically target ferritin receptors having high expression on cell surface which is a type of tumor marker highly expressed by various highly proliferated cells (e.g. tumor cells). Therefore, such cell targeting ability is broad-spectrum and can be used for targeting early diagnosis and treatment of various tumors;
4. this type of material not only can bind to cells highly expressing ferritin receptors, but also can enter into cells by endocytosis, and thus is a cell internalizing magnetic nano-material;
5. this type of material can be used as cell targeted magnetic resonance imaging contrast agent, fluorescent molecular probe and isotope marker for in vivo imaging early diagnosis of tumors. By using the material as magnetic resonance imaging contrast agent, early diagnosis of tumor with a size of about 1 mm is realized on animal models in the present invention.
6. this type of material can be used as a medicine vector for targeting treatment of tumors, realizing drug transportation within cells, capable of in vitro killing a broad spectrum of tumor cells and in vivo suppressing the tumors.

Further elaboration on the present invention will be made below in view of specific embodiments. It shall be construed that these embodiments are used only to explain the present invention but not to limit the scope of the present invention. In the following embodiments, any experiment with unspecified conditions is conducted under routine conditions, e.g. conditions described by Sambrook et al., Molecular Cloning: Laboratory Manual (New York: Cold Spring Harbor Laboratory Press, 1989), or under conditions recommended by the manufacturer. All percentage and proportion are counted by weight, unless otherwise specified.

The unit of weight volume percentage in the present invention is well known by one skilled in the art, which indicate, for example, the weight of solute in a 100 mL solution.

Unless otherwise defined, all professional and scientific terms used herein have the familiar meanings for one skilled in the art. In addition, any method and material similar or equivalent to the contents described herein can be used on the method of the present invention. The preferred embodiments and materials herein are used only for demonstration.

### Embodiment 1

### The biomimetic synthesis of human H-subunit magnetoferritin with complete protein-shell configuration

By using recombinant human ferritin as a template, the full length cDNA of human H-subunit human ferritin is cloned and constructed onto pET11b plasmid (purchased from Novagen Inc.); bacteria BL21 (DE3) pLysS (purchased from Novagen Inc.) are separately transformed or co-transformed by recombinant plasmids comprising human ferritin H-subunits and L-subunits. IPTG (isopropyl β-D-thiogalactopyranoside) is added to activate T7 promoter and induce expression. After expression, the proteins are released by ultrasonication, and then separated and purified. By using purified human H-subunit ferritin as a template, ferrous salts and the oxidant H₂O₂ are added into the solution of recombinant human ferritin, while the pH is controlled at 8.5 and the temperature is controlled at 65°C, and strongly magnetic nano-particles are thus formed inside the recombinant human ferritin. The concentration of ferrous salt is that the ratio of the number of ferrous atoms added each time to the number of protein molecules is 10-200, and the final number of iron atoms added into a single protein molecule is 5000. After reaction, human H-subunit magnetoferritin with complete protein-shell structure is obtained through size-exclusion chromatography, centrifugation and molecular sieve purification. FIG. 1a is the negatively stained transmission electron microscope (TEM) image of the obtained magnetoferritin, and each magnetic nano-core is coated with complete recombinant human H-subunit magnetoferritin. FIG. 1b is the transmission electron microscope image of the cores of obtained human magnetoferritins, which have uniform grain size and similar shapes, and show mono-dispersity. FIG. 1c is the histogram of the grain size distribution of human magnetoferritin, which indicates the narrow distribution of the grain size of the magnetic nano-cores, average 4.6 ±0.9 nm. FIG. 1d is the selected area electron diffraction image of human H-subunit magnetoferritin, which indicates its mineral phase is magnetite. FIG. 1e is the circular dichroism (CD) of the material, which indicates that the original configuration of ferritin shell is not destructed after biomimetic synthesis.

The amino acid sequence of the shell of human H-subunit ferritin of the material is as follows:

MTTASTSQVRQNYHQDSEAAINRQINLELYASYVYLSMSYYFDRDD
VALKNFAKYFLHQSHEEREHAEKLMKLQNQRGGRIFLQDIKKPDCDDWES
GLNAMECALHLEKNVNQSLLELHKLATDKNDPHLCDFIETHYLNEQVKAI
KELGDHVTNLRKMGAPESG LAEYLFDKHTLGDSDNES

### Embodiment 2

### Synthesis of the magnetic nano-material of manganese-iron oxide cores coated with ferritin shells

By using purified human H-subunit ferritin as a template, ferrous salt, manganese salt (the ratio of ferrous salt to manganese salt is 11.5, equivalent to 8% manganese) and the oxidant H₂O₂ are added into the solution of recombinant human ferritin, while the pH is controlled at 8.5 and the temperature is controlled at 65°C, and ferromagnetic nano-particles are thus formed inside the recombinant human ferritin, in which the number of iron atoms added in a single is eventually 4600, and the final number of manganese atoms added in a single is 400. After reaction, magnetic particles with complete protein structure are obtained through size-exclusion chromatography, centrifugation and molecular sieve purification. FIG. 2a is the scanning electron microscope image of the cores of manganese-iron oxide, which indicates that the formed cores are approximate sphere-shaped and showing excellent mono-dispersity. FIG. 2b is the distribution graph of the granularity of the cores, and the average grain size is 4.7 ±0.8 nm. FIG. 2c is the hysteresis loop of manganese-iron oxide measured at 2K. Compared with the human H-subunit magnetoferritin with cores consisting of magnetite (Fe₃O₄), the saturation magnetization of manganese-iron oxide is 23 emu/g, stronger than the original human H-subunit magnetoferritin (20 emu/g); its coercivity is 20 mT, while the coercivity of the original human H-subunit magnetoferritin is 37 mT at 2K. FIG. 2 is the element analysis by energy dispersive spectroscopy of the cores of manganese-iron oxide coated with ferritin shells, which indicates that there is manganese, iron and oxygen in the core, signifying manganese incorporated into the cores to form the magnetic nano-material of manganese-iron oxide (Mn_{0.24}Fe_{2.76}O₄).

### Embodiment 3

### Biomimetic synthesis of magnetic nano-materials by using membrane protein Mms6 prokaryotically expressed by magnetotactic bacteria

The complete genome of magnetotactic bacteria AMB-1 is extracted and the mms6 genes are amplified by PCR and cloned onto pET15b plasmid (purchased from Novagen Inc.) at EcoR I and BamH I restriction sites. The recombinant plasmid is transferred into BL21 (DE3) pLysS bacteria (purchased from Novagen Inc.) and expressed prokaryotically. Mms6 proteins labeled with His are purified by nickel affinity chromatography. His-Mms6 proteins and the solution of ferric salt are mixed, reacting for 24 hours at ambient temperature and pH 7-9 adjusted with NaOH, and ferrihydrite is produced therefrom. The solution of ferrous salt is added (to make the ratio of ferrous ions to ferric ions be 1:2) and the reaction is continued for another 24 hours until the solution turns completely black. The obtained magnetic particles are collected with magnets and washed 3 times with deoxygenated water. After freeze-drying, the magnetic particles are observed with electron microscope and magnetics measured. FIG. 3a is the image of prokaryotic expression and purification of Mms6 protein labeled with His. According to Track 2, with IPTG added, Escherichia coli are capable of expressing His-Mms6 proteins, whose molecular weight is about 10kD. Purified His-Mms6 proteins can be obtained through nickel affinity chromatography (Track 3). FIG 3b is the electron microscope image of magnetic nano-particles biomimetically synthesized by His-Mms6. The synthesized magnetic nano-particles have uniform grain size and similar shapes that approximate to sphere. FIG 3c is a high-resolution electron microscope image (crystal lattice) of magnetic nano-particles biomimetically synthesized by His-Mms6, which indicates that even at ambient temperature and pressure, the magnetic nano-particles biomimetically synthesized by His-Mms6 maintain perfect crystalline without defects of crystal lattice, and the crystalline is similar to the cubo-octahedral crystalline of the magnetosomes of magnetotatic bacteria. FIG. 3d is the X-ray crystallography (XRD) image of magnetic nano-particles biomimetically synthesized by His-Mms6, which, compared with the XRD peak image of the standard magnetite, indicates that the composition of the synthesized magnetic nano-particles is magnetite.

The amino acid sequence of the Mms6 protein shell of the material is as follows:

MGEMEREGAAAKAGAAKTGAAKTGTVAKTGIAAKTGVATAVAAP
AAPANVAAAQGAGTKVALGAGKAAAGAKVVGGTIWTGKGLGLGLGLGL
GAWGPIILGVVGAGAVYAYMKSRDIESAQSDEEVELRDALA

### Embodiment 4

### Targeting ability of human H-subunit magnetoferritin toward various cells that express ferritin receptors

Firstly, human H-subunit magnetoferritin is labeled with fluorescence by fluorescent stain Cy5.5 (that is, Cy5.5 is linked to human H-subunit magnetoferritin by covalent bond), and the fluorescent molecules unbound with proteins are removed by desalination column. When entering the logarithmic phase (about 60% of a culture bottle is occupied with cells), the cells in culture bottles are digested by pancreatic enzyme comprising 0.25% EDTA. The digested cells are washed three times with phosphate buffer solution (PBS, pH 7.4), and suspended with proper quantity of PBS added (the concentration of cells is about 1×10⁶ cell/ml). 100 µL cells suspended in PBS is taken out in a 1.5 mL centrifuge tube. 2 µL human H-subunit magnetoferritin (1 mg/mL) newly labeled with Cy5.5 is added to incubate with the cells in bath for 40 min. PBS of the same volume is added in the control group of the cells. After incubation, the materials unbound with cells are washed-out with PBS 3 times. In the end, 500 µL cells suspended in PBS is analyzed by quantitative fluorimetric analysis with flow cytometry BD FACS CantoTM Flow Cytometer. FIG. 4 is the image of flow analysis of various cells that express ferritin receptors. The result shows that, 10 of 11 cell lines can bind with specificity to human H-subunit magnetoferritin, which belong to mammary, glioma, hepatocellular and pulmonary carcinomas, indicating the material can bind to various tumor cells widely (the cells are purchased from ATCC, cultured at Nanjing KeyGEN BioTECH development Ltd.).

### Embodiment 5

### Cell targeting mechanism of human H-subunit magnetoferritin to the cells that express ferritin receptors in vitro

To research the mechanism of the interaction between the materials and the cells, the MDA-MB-231 cells that bind to the material at a high level are selected in the conduction of specific binding and competitive inhibition experiment with the materials. Before the Cy5.5 newly labeled human H-subunit magnetoferritin is added, the ferritin shells 100 times as many as said magneto ferritins are used for incubation 30 min in advance, and the binding with the material is observed. The cells are analyzed by quantitative fluorimetric analysis with BD FACS Canto™ flow cytometry. FIG. 5 is the result of flow analysis, which indicates that more than 50% of the binding between the material and MDA-MB-231 can be inhibited by ferritin shells 100 times in quantity, indicating the binding between the material and the cells is in dependent with the ferritin shells. In addition, more than 50% of the binding can also be inhibited by Anti-TfR1 antibody, which indicates the binding is mediated by TfR1. Besides, MX-1 cells, which bind to the materials at an extremely low level, is negative in the expression of TfR1, and this further confirms the specific, targeting interaction between human H-subunit ferritin and the cells that express ferritin receptors.

### Embodiment 6

### Measurement of transverse relaxation ratio (r₂) of human H-subunit magnetoferritin

Firstly, melt 1% agarose gel with low melt point (Sangon Biotech shanghai Co. Ltd) is used to dilute the material until the final iron concentration reaches 0-0.4 mM. Immediately after that, the material is frozen in a refrigerator at -20°C, and then the material is tested in an MRI system (Bruker, Biospin MRI PharmaScan 7.0T, 300 MHz, 1H model). Multi-slice multi-echo (MSME) series is used in T₂-weighted imaging of the measurement of r₂ of the material, and the specific parameters are: field of view: 3.5 cm, matrix : 256 × 256, repetition time: 5000 ms,1 echoes, TE: 11 ms, 1 section, slice thickness: 1 mm. The value of R₂, which equals to 1/T₂, is calculated Bruker Paravision 5.0, a software built in the computer. Then transverse relaxation ratio r₂ of the specimen is obtained by linear fitting of R₂ value with different iron concentrations. FIG. 6 shows the measured values of transverse relaxation (R₂) of human H-subunit magnetoferritin with different iron concentrations and a fitting curve thereof. It can be determined by fitting that in melt 1% agarose gel with low melt point, under 7T MRI condition the transverse relaxation ratio r₂ of the material is 54 mM⁻¹S⁻¹.

### Embodiment 7

### Targeting magnetic resonance imaging of the cells that express ferritin receptors in vitro

Firstly, about 10⁶ MDA-MB-231 cells that highly express ferritin receptors and MX-1 cells that lowly express ferritin receptors (purchased from ATCC, cultured at Nanjing KeyGEN BioTECH development Ltd.) are cultured in serum culture medium of a 6-well plate for 24 h. When the cells are almost completely attached to the plate, the old culture medium is removed and 1.5 mL fresh culture medium is added with the final concentration of human H-subunit magnetoferritin fixed at 165 µg/mL. The cells being cultured for 5.5 h the cells are washed 3 times with PBS. After being digested by 0.25% pancreatin comprising EDTA, the cells are washed another 3 times with PBS. The cells are put into a 96-well plate and diluted with PBS until the final concentration of the cells reaches 1×10⁶ cell/mL and the final volume of PBS is 100 µL, and then 100 µL 1% agarose gel (Sangon Biotech shanghai Co. Ltd) with low melt point is added. Immediately after that, the cells are frozen in a refrigerator at -20°C. The cells are put into a 7T MRI system especially for small animals (Bruker, Biospin MRI PharmaScan 7.0T, 300 MHz, 1H model) and magnetic resonance imaged using a rat body coil. FIG. 7 is the MRI image of the cells in vitro, which indicates that after incubation, the MRI signal intensity of TfR1-positive MDA-MB-231 cells is decreased significantly; while there is no significant change in the MRI signal intensity of TfR1-negative MX-1 cells. The result indicates the imaging is a molecular-targeted magnetic resonance imaging.

### Embodiment 8

### Verification of targeting ability of human H-subunit magnetoferritin to tissues that express ferritin receptors in in-vivo experiment

There are several barrier systems in vivo, and thus the environment is far more complicated in vivo than that in vitro. To verify whether human H-subunit ferritin targets tumors in vivo, MDA-MB-231 mammary carcinoma cells, which bind to human H-subunit magnetoferritin at a high level and is positive in TfR1 expression, and MX-1 mammary carcinoma cells, which bind to human H-subunit magnetoferritin at a low level and negatively express TfR1, are selected to establish nude mouse transplantation models. When the tumor grows to 2-3 mm, human H-subunit ferritins are injected through the tail vein (injection dosage: 10 mg Fe/kg weight of a mouse). The scanning is conducted prior to the injection of human H-subunit magnetoferritin (Pre), 1.5h, 3.5 h and 5.5 h after injection, respectively. Multi-slice multi-echo (MSME) sequence is used in T₂-weighted imaging with the following parameters: field of view (FOV) = 3.5 cm × 3.5 cm, matrix = 256 × 256, repetition time (TR) = 3,000 ms, 6 echoes with echo time (TE) = 15, 30, 45, 60, 75, 90 ms, 20 slices, slice thickness 0.8 mm. Multi-gradient echo (MGE) sequence is used in T₂*-weighted imaging with the following parameters: FOV = 3.5 cm × 3.5 cm, matrix = 256 × 256, TR = 900 ms, 6 echoes with TE = 4, 10, 16, 22, 28, 34 ms, 20 slices, slice thickness 0.8 mm. MRI image processing is conducted using the built-in software Bruker Paravision 4.0. The signal intensity of the tissues is quantified by signal-to-noise ratio (SNR). SNR=SIₜᵤₘₒᵣ/SD_{muscle}, SIₜᵤₘₒᵣ is the average signal intensity of carcinoma lesion, SD_{muscle} is the standard deviation of the average signal intensity of the muscles near the carcinoma lesion. Tissue contrast enhancement (CE) at a time t after the injection of human H-subunit magnetoferritin is calculated through the following formula: CE (%) = (SNRₚᵣₑ - SNRₜ) / SNRₚᵣₑ [Buerke et al., 2008; Tsurusaki et al., 2008]. FIGS. 8a-c are T₂* MRI images of an MDA-MB-231 tumor borne mouse, a ferritin competitive inhibited MDA-MB-231 tumor borne mouse and an MX-1 tumor borne mouse, indicating that after human H-subunit magneto ferritins are injected, there is a significant change in the signal intensity of MDA-MB-231 tumor that highly expresses TfR1 (n=5), while the change is small in the signal intensity of MDA-MB-231 tumor saturated with ferritin shell receptors (n=4), and the change is also small in the signal intensity of MX-1 tumor that lowly expresses TfR (n=5). Thus, it indicates that human H-subunit magnetoferritin is an MRI contrast agent with specific, active tissue targeting that depends on TfR1-expressed tissue. FIG. 8d is the quantitative analysis of T₂*-weighted images. Relative contrast enhancement of MDA-MB-231 carcinoma lesion prior to injection (Pre), 1.5h, 3.5h and 5.5h after injection are 41.4 ± 11.8 % (average ± standard deviation), 59.0 ± 5.5 % and 56.6 ± 5.5 %, respectively. FIG. 8e is the image of tumor paraffin slices from the mice sacrificed immediately after MRI scanning. The histological result shows that, after stained with DAB enhanced Prussian blue, MDA-MB-231 carcinoma lesion shows evident staining positivity (brown particles), indicating that a massive amount of iron particles aggregate in MDA-MB-231 carcinoma lesion. However, there is no positive staining with specificity shown in MDA-MB-231 carcinoma lesion competitively inhibited by human H-subunit ferritin and MX-1 carcinoma lesion, indicating no or little aggregation of the material. The result of iron staining in histology corresponds in highly degree to the MRI result.

**Table 1 SNR of T₂ and T₂* MRI images of TfR1-positive MDA-MB-231 mammary carcinoma cells (n=5), MDA-MB-231 tumor inhibited by human ferritin shells (n=4) and MX-1 mammary carcinoma cells (n=5) prior to and after human H-subunit ferritin injected**

| Tumor group | time | SNR (T₂ MRI image) | *P* value (comparison between prior to and after injection) | SNR (T₂* MRI image) | *P* value (comparison between prior to and after injection) |
|---|---|---|---|---|---|
| MDA-MB-231 tumor | Prior to injection | 19.17 ± 4.4 | | 22.18 ± 4.5 | |
| | After injection | | | | |
| | 1.5 h | 15.96 ± 4.9 | 0.031 | 13.23 ± 4.4 | 0.031 |
| | 3.5 h | 14.64 ± 4.2 | 0.031 | 8.92 ± 1.2 | 0.031 |
| | 5.5 h | 14.89 ± 4.6 | 0.031 | 9.68 ± 2.5 | 0.031 |
| shells MDA-MB-231 tumor inhibited by human ferritin | Prior to injection | 19.06 ± 3.8 | | 17.23 ± 2.9 | |
| | After injection | | | | |
| | 1.5 h | 18.88 ± 3.9 | 0.438 | 16.37 ± 3.7 | 0.125 |
| | 3.5 h | 18.97 ± 3.4 | 0.563 | 13.6 ± 4.2 | 0.063 |
| | 5.5 h | 18.88 ± 3.5 | 0.563 | 12.72 ± 3.2 | 0.063 |
| MX-1 tumor | Prior to injection | 19.55 ± 3.0 | | 23.81 ± 2.2 | |
| | After injection | | | | |
| | 1.5 h | 19.54 ± 2.3 | 0.500 | 23.19 ± 3.0 | 0.219 |
| | 3.5 h | 18.83 ± 1.8 | 0.156 | 22.18 ± 2.3 | 0.031 |
| | 5.5 h | 18.71 ± 2.5 | 0.156 | 21.14 ± 3.1 | 0.031 |

| | | | | | |
|---|---|---|---|---|---|
| Note: SNR is in the form of average ± s.d. Significance difference is tested with Wilcoxon test. P < 0.05 indicates the significance difference is obvious. | | | | | |

### Embodiment 9

### Molecular mechanism of human H-subunit magnetoferritin targeting in vivo tumor

To research the mechanism of human H-subunit magnetoferritin targeting tissues with specificity, we conducted in vivo fluorescent tracing experiment and in vitro immunity fluorescence experiment. Firstly, human H-subunit magnetoferritin is labeled with fluorescent stain rhodamine B, and the labeled materials are injected through tail veins of the nude mice bearing MDA-MB-231 tumor (n=3) and the nude mice bearing MX-1 tumor (n=3), respectively. After being cultured in a dark ambient absent of light for 3 h, the mice are perfused with PBS into hearts, and the carcinoma lesion tissues are immediately dissected out, wrapped in tin foils and kept overnight in liquid nitrogen in the absence of light. The tissue is embedded with OCT (optimum cutting temperature compound, Sakura), and sliced on Leica cryostat (5µm in thickness). After OCT is removed by drying, the slices are put in acetone to immobilize for 15 min. Following drying, the slices are stored in a refrigerator at -80°C. To conduct fluorescent observation, the slices are washed 3 times, and then 10% bovine serum albumin (BSA) is added and incubated for 30 min at 37 °C to avoid fluorescent adsorption without specificity. After that, the slices are stained (37 °C, 1.5 h) with anti-TfR1 labeled with FITC. Anti-TfR1 adsorbed without specificity is washed off with PBS, and the slices are mounted with anti-quenching mounting medium. FIG. 9 is the staining result of in vivo fluorescent tracing experiment and in vitro immunity fluorescence experiment. The result shows that, the red fluorescence of human H-subunit magnetoferritin overlaps well with the green fluorescence of anti-TfR1, indicating the molecular mechanism of human H-subunit magnetoferritin targeting tissues with specificity is in dependence on the binding to the TfR1 expressed on tissues.

### Embodiment 10

### Human H-subunit magnetoferritin used in early diagnosis of minute mammary carcinoma

When MDA-MB-231 tumor in a nude mouse grows to about 1 mm, human H-subunit magnetoferritin is injected through tail vein (injection dosage: 10mg Fe/kg weight of mouse). The scanning is conducted prior to the injection (Pre) and 5.5 h after the injection. Multi-section multi-echo (MSME) sequence is used in T₂-weighted imaging with the following parameters: field of view (FOV) = 3.5 cm × 3.5 cm, matrix = 256 × 256, repetition time (TR) = 3,000 ms, 6 echoes with echo time (TE) = 15, 30, 45, 60, 75, 90 ms, 20 slices, slice thickness 0.8 mm. Multi-gradient echo (MGE) sequence is used in T₂*-weighted imaging with the following parameters: FOV = 3.5 cm × 3.5 cm, matrix = 256 × 256, TR = 900 ms, 6 echoes with TE = 4, 10, 16, 22, 28, 34 ms, 20 slices, slice thickness 0.8 mm. MRI image processing is conducted using the built-in software Bruker Paravision 5.0.

FIGS. 10a and b are T₂*-weighted MRI images of nude mice bearing MDA-MB-231 minute carcinoma lesion. The images show that, after human H-subunit magneto ferritins are injected, the signal intensity of carcinoma lesion changes significantly; the signal intensity after injection is much weaker than that before injection, and the carcinoma lesion gets darker in the image. After quantitative and statistical analysis of T₂*-weighted image, there is a significant difference in SNR value prior to and after human H-subunit ferritin is injected (FIG. 10c). In T₂-weighted MRI image, the signal intensity does not change significantly, but it can be indicated by quantitative analysis that there is significant difference in SNR prior to and after the injection of the materials (FIGS. 10d-f). After being stained with DAB enhanced Prussian blue, brown particles appear in paraffin slices of carcinoma lesion tissue, showing an obviously positive result. This indicates that human H-subunit magneto ferritins have already aggregated in minute carcinoma lesion (FIG. 10g). FIG. 10h is the photo of MDA-MB-231 minute carcinoma lesion dissected out, which shows the diameter of the tested carcinoma lesion is about 1 mm. The carcinoma lesion is weighed with balance, and it weighs about 2 mg.

### Embodiment 11

### Human H-subunit magnetoferritin used in early diagnosis of tumor as fluorescent molecular probe

Human H-subunit magnetoferritin is labeled with near-infrared stain Cy5.5 (that is, Cy5.5 is linked to human H-subunit magnetoferritin by covalent bonds), and thus becomes a tumor targeted fluorescent molecular probe. When MDA-MB-231 tumor grows to about 3 mm, human H-subunit magneto ferritins labeled with Cy5.5 are injected through tail vein, and then scanned with in vivo fluorescence imaging system CRI Metro ™ to observe its relative in vivo distribution. FIG. 11 is the in vivo fluorescence image 3 h after human H-subunit magneto ferritins are injected. Besides strong fluorescent intensity distributed outside the liver and the bladder, there is also relatively strong fluorescent intensity distributed in the tumor area obviously.

### Embodiment 12

### Human H-subunit magnetoferritin used in early diagnosis of minute hepatocellular carcinoma

TfR1-positive QGY7701 hepatocellular carcinoma cells (purchased from ATCC, cultured at Nanjing KeyGEN BioTECH development Ltd.), are used to establish nude mouse hepatocellular carcinoma transplantation models. When the tumor in a nude mouse grows as big as ∼1mm, human H-subunit magnetoferritin is injected through tail vein (injection dosage: 10mg Fe/kg weight of mouse). The scanning is conducted prior to the injection (Pre) and 5.5 h after the injection. Multi-section multi-echo (MSME) sequence is used in T₂-weighted imaging with the following parameters: field of view (FOV) = 3.5 cm × 3.5 cm, matrix = 256 × 256, repetition time (TR) = 3,000 ms, 6 echoes with echo time (TE) = 15, 30, 45, 60, 75, 90 ms, 20 slices, slice thickness 0.8 mm. Multi-gradient echo (MGE) sequence is used in T₂*-weighted imaging with the following parameters: FOV = 3.5 cm × 3.5 cm, matrix = 256 × 256, TR = 900 ms, 6 echoes with TE = 4, 10, 16, 22, 28, 34 ms, 20 slices, slice thickness 0.8 mm. MRI image processing is conducted using the built-in software Bruker Paravision 5.0. The signal intensity of the carcinoma lesion is quantified with signal-to-noise ratio (SNR). The result is shown in FIG. 12. The result indicates that in T₂* image, there is a significant change in the signal intensity of the tumor (red circle) comparing with that before injection.

### Embodiment 13

### Human H-subunit magnetoferritin used in early diagnosis of minute pulmonary carcinoma

TfR1-positive NCI-H460 human pulmonary carcinoma cells (purchased from ATCC, cultured at Nanjing KeyGEN BioTECH development Ltd.) are used to establish nude mouse human pulmonary carcinoma transplantation models. Human H-subunit magnetoferritin is injected through tail vein (injection dosage: 10mg Fe/kg weight of mouse). The scanning is conducted prior to the injection (Pre) and 5.5 h after the injection. Multi-section multi-echo (MSME) sequence is used in T₂-weighted imaging with the following parameters: field of view (FOV) = 3.5 cm × 3.5 cm, matrix = 256 × 256, repetition time (TR) = 3,000 ms, 6 echoes with echo time (TE) = 15, 30, 45, 60, 75, 90 ms, 20 slices, slice thickness 0.8 mm. Multi-gradient echo (MGE) sequence is used in T₂*-weighted imaging with the following parameters: FOV = 3.5 cm × 3.5 cm, matrix = 256 × 256, TR = 900 ms, 6 echoes with TE = 4, 10, 16, 22, 28, 34 ms, 20 slices, slice thickness 0.8 mm. MRI image processing is conducted using the built-in software Bruker Paravision 5.0. The signal intensity of the carcinoma lesion is quantified with signal-to-noise ratio (SNR). The result is shown in FIG. 13. The result indicates that in T₂* image, there is a significant change in the signal intensity of the tumor (red circle) comparing with that before injection.

### Embodiment 14

### The distribution of human H-subunit magnetoferritin in tissues and organs in a nude mouse

A nude mouse bearing MDA-MB-231 (purchased from ATCC, cultured at Nanjing KeyGEN BioTECH development Ltd.) injected with the materials (10 mg Fe/kg weight) is sacrificed 6 h after injection, and its muscle, heart, liver, pancreas, kidney, brain, axillary lymph node and tumor are dissected out for paraffin slice processing. The distribution of human H-subunit magnetoferritin in tissues and organs is tested with DAB enhanced Prussian blue. Firstly, the paraffin slices are dried in an oven at 60°C for 1 h, soaked twice in xylene at 37°C (15 min each time) to dewax. Then the paraffin slices are dehydrated twice with 100% alcohol (5 min each time), and then rehydrated in 80% alcohol and deionized water (2 min each time). To remove inherent peroxide enzymes, the slices, after rehydration, are put into methanol solution comprising 3% H₂O₂ and treated for 30 min, and then washed with deionized water 3 times. The steps of iron staining are: firstly staining the slice with Prussian blue (freshly prepared with 10% potassium ferrocyanide and 20% hydrochloric acid) for 20 min, and then staining it with PBS comprising 0.033% H₂O₂ and 0.05% DAB for 15 min; finally using hematoxylin and eosin solutions to stain nucleus and cytoplasms. FIG. 14 shows the iron staining photos of the mouse's muscle, heart, liver, pancreas, kidney, brain, axillary lymph node and tumor after the injection of human H-subunit magnetoferritin, which shows evident staining positivity; after staining, there is a large quantity of brown particles shown in the tissues, indicating a massive amount of iron particles of the materials distributed in the tissues; there is a positive staining, to some degree, in the liver, and there are iron particles distributed in Kupffer cells; there are iron particles existing obviously between the cortex and the medulla of the lymph node; while the pancreas, heart, lung, kidney, brain and muscle tissues show negative staining, indicating there are few materials distributed in the tissues.

### Embodiment 15

### Biological distribution of the materials in tumor tissues and cells

To observe the distribution of human H-subunit magnetoferritin in cancer tissues and cells, the nude mice bearing MDA-MB-231 mammary carcinoma are injected with human H-subunit magnetoferritin, and sacrificed 24 h later. The carcinoma lesions are immediately dissected out and sliced to 1 mm³, and then immobilized in 2.5% glutaraldehyde at 4°C. The preparation steps of TEM ultra-thin slices are as follows: taking out the glutaraldehyde immobilized tissues and wash them with PBS (0.01M, pH 7.4) three times (10 min each time); using osmate acid to immobilize the tissues for 25 min (during the immobilization process, it should be observed whether the tissues turn black at about 20 min); washing the tissues with PBS once, and double distilled water twice (10 min each time); staining the tissues with 1% uranyl acetate for 1 h, and then use 50%, 70%, 85% and 95% ethanol to dehydrate, 12 min each time, and then using 100% ethanol to dehydrate three times, 15 min each time; using ethanol and epoxy resin investment liquid to immerse the tissues three times, 2 h each time (the ratio of ethanol to embedding solution is 1:1), and then using pure epoxy resin embedding solution to immerse the tissues overnight. 2 h after replacing the embedding solution with a new pure embedding solution, embed the tissues (in a dry oven at 60°C, 24 h). The ultra-thin slices are observed with JEM-1400 transmission electron microscope, under 120 kV accelerating voltage. FIG. 15 is the photo of TEM ultra-thin slice of the tumor tissues injected with the materials, which shows that a massive amount of iron particles with high electron density appear in cancer cells. This indicates that a large quantity of cancer cells have entered human H-subunit magnetoferritin, while there is no iron particle with high electron density observed in lymph cells and macrophage cells of the tumor tissues. The distribution of the materials in tumor tissues brings an opportunity for tumor treatment.

### Embodiment 16

### Cytotoxicity experiment of doxorubicin linked human H-subunit magnetoferritin to tumor cells

Doxorubicin hydrochloride is cross-linked to human H-subunit magnetoferritin with glutaraldehyde, and the unlinked doxorubicin hydrochloride is removed with G50 desalination column. By the use of a spectrophotometry, the protein concentration of human H-subunit magnetoferritin (BCA method) and the concentration of doxorubicin (485 nm) are measured respectively, and it is determined that about every 48 molecules of doxorubicin link to one material. FIG. 16a is the photo showing the color changes of the materials after linked to doxorubicin, which shows that the material turns to dark red from dark brown after linked to doxorubicin.

When the growth of tumor cells enters logarithmic phase, 100 µL tumor cells is added in an amount of 5000 cells per well to a 96-well, and the volume of each well was 100 µL. Marginal wells are filled with sterile PBS. The cells are cultured with the doxorubicin linked materials at different concentrations of doxorubicin (1-20000nM, 11 concentrations in gradient) in a 37°C incubator with 5% CO₂ for 72 h. With 20 µL 0.5% MTT added, the culture of the cells is continued for 4 h, and then culture solution is removed by adsorption carefully. Each well is added with 150 µL dimethyl sulfoxide, and the plate is shaken at a low speed in a shaking table for 10 min so that the crystal is sufficiently dissolved. The absorbance of each well is measured by enzyme linked immunosorbent assay, OD at 490 nm. Meanwhile, zero setting wells (culture medium, MTT and DMSO), and control wells (cells, drug dissolvent medium at the same concentration, culture solution, MTT and DMSO) are also set. FIG. 16b is the result of cytotoxicity experiment of doxorubicin linked materials to liver cancer cells QGY7701, leukemia cancer cells K562, glioma cells U87MG, lung cancer cells NCI-H460, rectum cancer cells HT-29, mammary carcinoma cells MDA-MB-231 (purchased from ATCC, cultured at Nanjing KeyGEN BioTECH development Ltd.), and it is found that the doxorubicin linked materials have obvious toxicity to these cells.

### Embodiment 17

### Treatment experiment on lung cancer in vivo using doxorubicin linked human H-subunit magnetoferritin

NCI-H460 lung cancer cells, which express ferritin receptors at a high level, are chosen to be subcutaneously transplanted into the axillary region of each nude mouse. When the tumor grows to about 1 cm, the nude mice are divided into 3 groups to administer drugs for treatment: PBS control group, doxorubicin hydrochloride treated group and Doxorubicin-linked materials treated group, 3 mice per group. The mice are administered drugs every 3 days, and the concentration of administered doxorubicin is 3 mg/kg weight. Before administration of the drugs, the width and length of the tumors are measured with vernier caliper. 15 days later after administration of the drugs, the mice are sacrificed by cervical dislocation, and then the tumors are dissected out after anatomizing the mice, weighed and photographed. FIG. 17a is a graph of the volumes of tumors versus days after being injected with the drugs. 8 days after the administration of the drugs, there is a significant difference between the Doxorubicin-linked materials treated group and the control group, and minute difference between the doxorubicin hydrochloride treated group and the control group, showing non-obvious effect of treatment. FIG. 17b shows the weights of the tumors after being dissected out. The average tumor inhibition rates of doxorubicin linked materials treated group and doxorubicin hydrochloride treated group are 39% and 23% respectively. According to FIG. 17c, the photo of the tumors, compared with PBS control group, Doxorubicin-linked materials show obvious effects on tumor inhibition.

### Embodiment 18

### Biomimetic synthesis of magnetoferritin with various grain sizes and transverse relaxation rates (r₂)

By using purified human H-subunit ferritin as a template (protein concentration 0.5-1 mg/mL), a ferrous salt and oxidant H₂O₂ are added into a solution of recombinant human ferritin, while the pH is controlled at 8-9 and the temperature is controlled at 60-80°C. By precisely controlling the ratio of ferrous ions to proteins and precisely controlling the temperature and pH to be constant, the final number of iron atoms added into a single protein molecule is 1000, 3000, 5000, 7000 and 10000, respectively; after the reaction is completed, mono-dispersed magnetoferritin particles with a complete protein structure is obtained through size-exclusion chromatography, centrifugation and molecular sieve purification. The relaxation time is measured with a 4.7T MRI system (Bruker) in PBS solution at ambient temperature using multi-slice multi-echo (MSME) sequence with the following parameters: field of view (FOV) = 5 cm × 5 cm, matrix = 196 × 196, repetition time (TR) = 3000 ms, 10 echoes with echo time (TE) = 8.5, 17, 25.5, 34, 42.5, 51, 59.5, 68, 76.5, 85 ms.

FIGS. 18a, 18b, 18c and 18d show the transmission electron microscope (TEM) photo, the histogram of grain size distribution, the transverse relaxation rate (r₂) and the low temperature (5K) magnetic hysteresis loop, respectively, of the magnetoferritin synthesized in the reaction in which an average of 1000 iron atoms are added into a single protein molecule. It can be seen from the graphs that the average grain size is 2.7 ± 0.6 nm, and the value of r₂ is 23 mM⁻¹s⁻¹. FIGS. 18e, 18f, 18g and 18h show the transmission electron microscope (TEM) photo, the histogram of grain size distribution, the transverse relaxation rate (r₂) and the low temperature (5K) magnetic hysteresis loop, respectively, of the magnetoferritin synthesized in the reaction in which an average of 3000 iron atoms are added into a single protein molecule. It can be seen from the graphs that the average grain size is 3.3 ± 0.8 nm, and the value of r₂ is 63 mM⁻¹s⁻¹. FIGS. 18i, 18j, 18k and 18l show the transmission electron microscope (TEM) photo, the histogram of grain size distribution, the transverse relaxation rate (r₂) and the low temperature (5K) magnetic hysteresis loop, respectively, of the magnetoferritin synthesized in the reaction in which an average of 5000 iron atoms are added into a single protein molecule. It can be seen from the graphs that the average grain size is 5.2 ± 1.0 nm, wherein the value of r₂ is 224 mM⁻¹s⁻¹. FIGS. 18m, 18n, 18o and 18p show the transmission electron microscope (TEM) photo, the histogram of grain size distribution, the transverse relaxation rate (r₂) and the low temperature (5K) magnetic hysteresis loop, respectively, of the magnetoferritin synthesized in the reaction in which an average of 7000 iron atoms are added into a single protein molecule. It can be seen from the graphs that the average grain size is 5.4 ± 1.1 nm, and the value of r₂ is 321 mM⁻¹s⁻¹. FIGS. 18q, 18r, and 18s show the transmission electron microscope (TEM) photo, the histogram of grain size distribution and the low temperature (5K) magnetic hysteresis loop, respectively, of the magnetoferritin synthesized in the reaction in which an average of 10000 iron atoms are added into a single protein molecule. It can be seen form the graphs that the average grain size is 7.1 ± 1.4 nm. It can be seen from the low temperature (5K) magnetic hysteresis loops of magneto ferritins with various granularities shown in FIGS. 18h, 18l, 18p and 18s, that saturation magnetization Ms rises significantly with increasing grain size, the saturation magnetizations being 5.9 Am²/kg (total mass) (1000), 15.2 Am²/kg (3000), 28.6 Am²/kg (5000), 37.1 Am²/kg (7000), 51.8 Am²/kg (10000), respectively. The magnetic hysteresis loops for all core grain sizes reach saturation at a magnetic field intensity lower than 1T, which indicates these ferritins are soft magnetic ferromagnetic mineral. Such property is totally different from that of previously reported ferritin (Uchida et al., 2006, Figure 7) that it still cannot reach saturation even at 8T (80000 Oe). Under the condition of same core grain size, the saturation magnetization of the magnetic particles of the present invention is 4 times that of the previously reported magnetic particles, and the transverse relaxation rate r₂ is more than 3 times that of the previously reported magnetic particles (Uchida et al., 2006; 2008). FIG. 18t shows the electron energy loss spectroscopy (EELS) of the magnetoferritin synthesized by adding an average of 5000 iron atoms into a single protein molecule (average grain size 5.2nm), wherein the L2 peak is at 708 eV and the L3 peak is at 722 eV, which indicates magnetite particles with standard stoichiometry, compared with the standard spectrum of a magnetite. As for the electron energy loss spectroscopy (EELS) of the previously reported magnetic nano-particles synthesized with ferritin, however, the L2 peak is at 704 eV and the L3 peak is at 715 eV (Uchida et al., 2006). Therefore, in a general view of the material science evaluation results for transverse relaxation rate measurement, low temperature magnetic hysteresis loops and electron energy loss spectroscopy etc., the magnetoferritin of the present invention has a different mineral phase than the previously reported magnetoferritin nano-particles, thus having typical distinctions on the magnetic properties and magnetic resonance relaxation effect.

### Embodiment 19

### Use of magnetoferritin with high relaxation rate (r₂) in mammary carcinoma molecular imaging

By using the magnetoferritin synthesized in the reaction in which an average of 5000 iron atoms are added into a single protein molecule as contrast agent, TfR1-positive MDA-MB-231 mammary carcinoma cells (tumor borne on the right posterior back) and TfR1-negative MX-1 mammary carcinoma cells (tumor borne on the left posterior back) are selected to establish nude mouse transplantation models. When the tumor grows to 2-3 mm, the nude mouse is anesthetized by respiratory anesthesia and an intravenous needle is detained in tail vein. The mouse is put into a 4.7T MRI system and stays in stationary state in the course of magnetic resonance scanning, which ensures the matching of the magnetic resonance scanning photos. Human H-subunit magneto ferritins are injected through the tail vein (injection dosage: 20 mg Fe/kg mouse weight). The scanning is conducted prior to the injection of human H-subunit magnetoferritin (0 h) and 6 hours after injection. Multi-slice multi-echo (MSME) sequence is used in T₂-weighted imaging for 26 min with the following parameters: FOV = 4 cm × 4 cm, matrix = 256 × 256, TR = 3000 ms, TE = 16, 32, 48, 64, 80, 96 ms, 20 slices, slice thickness 0.80 mm. Multi-gradient echo (MGE) sequence is used in T₂*-weighted imaging, with the following parameters: FOV = 4 cm × 4 cm, matrix = 256 × 256, TR = 950 ms, 6 echoes with TE = 4.5, 11.95, 19.4, 26.85, 34.3, 41.75 ms, 20 slices, slice thickness 0.80 mm. MR image processing is conducted using the built-in Bruker Paravision 4.0. Tumor MR signal changes are analyzed using the ratio (TNR) of tumor signal intensity to surrounding normal muscle signal intensity. TNR = SIₜᵤₘₒᵣ / SI_{muscle}, wherein SIₜᵤₘₒᵣ is the average signal intensity of the tumor area, and SI_{muscle} is the average signal intensity of normal muscles. The relative TNR reduction is calculated through the following formula: TNR reduction (%) = (TNRₚᵣₑ - TNRₜ) / (TNRₚᵣₑ).

FIGS. 19a-d show the T₂-weighted MRI images of a nude mouse with a tumor of about 3 mm. FIGS. 19e-h show the T₂*-weighted MRI images of a nude mouse with a tumor of about 3 mm. The results show that the signal intensity of TfR1-highly-expressed MDA-MB-231 tumor changes significantly after the injection of human H-subunit magnetoferritin, while the signal intensity of TfR1-negative MX-1 tumor does not show a notable change, thus indicating that the in vivo tissue targeted human H-subunit magnetoferritin is a specific and TfR1-depending tissue-actively-targeted MRI contrast agent. FIGS. 19i and 19j are the in-situ stereoscopic microscope photos of the tumor. It can be seen that the tumor size is about 3 mm. FIGS. 19k and 19l show the quantitative analysis of the T₂-weighted images and the T₂*-weighted images (statistics of 4 models). The TNR reduction of MDA-MB-231 tumor is significantly different from that of MX-1 tumor (P = 0.029). FIGS. 19m and 19n are the immunohistochemistry images of the tumors after magnetic resonance scanning. It can be seen that through DAB enhanced Prussian blue staining, MDA-MB-231 tumor tissues show evident staining positivity (brown particles), which indicates that a massive amount of iron particles aggregate in the MDA-MB-231 tumor. The iron rich tissue area corresponds in a high degree to the TfR1-highly-expressed tumor tissue, which indicates that magnetoferritin is a specific molecule targeted magnetic resonance contrast agent. Meanwhile, the MX-1 tumor tissue shows no specific positive staining, which indicates none or little aggregation of the material. The histological iron staining result corresponds in a high degree to the MRI result, which indicates that magnetoferritin is a targeting molecular probe that can be used to monitor the molecular imaging of the in vivo expression of TfR1.

### Embodiment 20

### Transverse relaxation rate (r2) magnetoferritin used in early MRI diagnosis of minute mammary carcinoma

By using the magnetoferritin synthesized in the reaction in which an average of 5000 iron atoms are added into a single protein molecule in embodiment 18 as contrast agent, TfR1-positive MDA-MB-231 mammary carcinoma cells are selected to establish nude mouse transplantation models. When the tumor grows to about 1 mm, the nude mouse is anesthetized by respiratory anesthesia and an intravenous needle is detained in tail vein. The mouse is put into a 4.7T MRI system and stays in stationary state in the course of magnetic resonance scanning, which ensures the matching of the magnetic resonance scanning photos. Human H-subunit magneto ferritins are injected through the tail vein (injection dosage: 20 mg Fe/kg mouse weight). The scanning is conducted prior to the injection of human H-subunit magneto ferritin (0 h) and 6 hours after injection. For T₂-weighted imaging, multi-slice multi-echo (MSME) sequence is used for 26 min; for T₂*-weighted imaging, multi-gradient echo (MGE) sequence is used. The sequence parameters are the same as those in embodiment 19. MR image processing is conducted using the built-in Bruker Paravision 4.0. Tumor MR signal changes are analyzed using the ratio (TNR) of tumor signal intensity to surrounding normal muscle signal intensity. TNR = SIₜᵤₘₒᵣ / SI_{muscle}, wherein SIₜᵤₘₒᵣ is the average signal intensity of the tumor area, and SI_{muscle} is the average signal intensity of normal muscles. The relative TNR reduction is calculated through the following formula: TNR reduction (%) = (TNRₚᵣₑ - TNRₜ) / (TNRₚᵣₑ).

FIGS. 20a-d show the T₂-weighted MRI images of a nude mouse with a tumor of about 1 mm. FIGS. 20e-h show the T₂*-weighted MRI images of a nude mouse with a tumor of about 1 mm. The results show that the signal intensity of the minute tumor changes significantly after the injection of human H-subunit magneto ferritin. Particularly, a naked eye discernible black area appears in the T₂*-weighted images. FIGS. 20i and 20j show the quantitative analyses of the T₂-weighted images and the T₂*-weighted images. It can be seen that the TNR of the minute tumor decreases significantly after the injection of the magneto ferritin. FIG. 20k is the in-situ stereoscopic microscope photo of a subcutaneous transplantation mammary carcinoma. The size of the tumor is about 0.6 mm. FIG. 20l shows the immunohistochemistry of the tumor after magnetic resonance scanning. It can be seen that the minute tumor with a size smaller than 1 mm shows no evident angiogenesis (CD31 staining negative), but a massive amount of magnetoferritin particles can aggregate in the minute tumor (DAB enhanced Prussian blue staining notably positive), indicating that magnetoferritin particles may have the function of penetrating vascular endothelial cell barrier.

### Embodiment 21

### Ferritin used as fluorescent molecular probe in early diagnosis of minute mammary carcinoma

Purified human H-subunit ferritin and NHs-Cy5.5 fluorescent molecules, according to the proportion of 24 molecules to a single protein, are incubated overnight, such that about 6-7 Cy5.5 fluorescent molecules are linked to a ferritin shell (fluorescence spectrophotometer is used to identify the number of fluorescent molecules linked). Then the ferritins are injected through tail vein into a nude mouse mammary carcinoma transplantation model with a tumor size of about 1mm. A TfR1-negative MX-1 tumor is borne on the left flank of the nude mouse model, while a TfR1-highly-expressed MDA-MB-231 tumor is borne on the right. Imaging is conducted using a near infrared fluorescence in vivo imaging system (CRI Maestro) at prior to injection, 1.5h, 3h, and 6h after injection, respectively. Image processing is conducted using the built-in software of the imaging system, with the same nude mouse prior to the injection of Cy5.5-ferritin as reference.

FIGS. 21a-21d are the fluorescence imaging photos prior to the injection of Cy5.5 ferritin and 1.5h, 3h, and 6h after injection. The images evidently show that after the injection of Cy5.5 ferritin, the TfR1-highly-expressed MDA-MB-231 minute tumor area shows intensive fluorescence, and thus can be clearly distinguished from the skin and muscle background. Meanwhile, the fluorescence imaging of the TfR1-negative MX-1 minute tumor cannot be distinguished from the skin and muscle background. FIG. 21e is the white light photo of the MX-1 tumor, MDA-MB-231 tumor and surrounding normal muscle tissues dissected out. It can be seen from the scale that the tumor size is about 1 mm. FIG. 21f is the fluorescence photo of the tissues dissected out. The fluorescence intensity of MDA-MB-231 tumor is evidently greater than that of the MX-1 tumor and the normal muscle tissues, indicating that in the presence of muscle fluorescence background, Cy5.5 ferritin fluorescent molecular probe can be used in near infrared diagnosis of TfR1-highly-expressed minute mammary carcinoma.

### Embodiment 22

### Ferritin used as fluorescent molecular probe in near infrared fluorescence in vivo imaging of pancreatic carcinoma

Cy5.5-linked human H-subunit ferritins are injected through tail vein into a nude mouse pancreatic carcinoma transplantation model with a tumor size of about 3 mm. the tumor cells are CFPAC-1 pancreatic carcinoma cell lines (purchased from ATCC, cultured at Nanjing KeyGEN BioTECH development Ltd.). Imaging is conducted using a near infrared fluorescence in vivo imaging system (CRI Maestro) prior to injection, 1.5h, 6h, 24h, 72h and 96h after injection.

FIG. 22 is image of the Cy5.5 ferritin probe injected near infrared fluorescence in vivo imaging photo, and the fluorescence imaging photos of individual tissue or organ (heart, liver, spleen, lung, kidney, brain, stomach, intestine, bone, tumor and muscle) dissected out after 96h. It can be known from the photos that the human H-subunit ferritins exist primarily in the liver and spleen after 96h. However, considering that fluorescence intensity is influenced by the size of surface area, and the surface area of liver is far larger than that of spleen, the specific distribution amounts of the ferritin needs to be verified by further measuring the fluorescence intensity of tissue lysate per unit weight.

### Embodiment 23

### Magnetoferritin used in early MRI diagnosis of in-situ glioma

10⁶ U87MG human glioma cells (purchased from ATCC, cultivated at Nanjing KeyGEN BioTECH development Ltd.) are inoculated onto the cerebral cortex of a nude mouse by in-situ penetration through the cranium and its tympanic membrane slightly off the median line of the cerebrum. Magnetic resonance imaging is conducted after about 3-4 days. The nude mouse is anesthetized by respiratory anesthesia and an intravenous needle is detained in tail vein. The mouse is put into a 4.7T MRI system and stays in stationary state in the course of magnetic resonance scanning. The commercialized Gd-DTPA contrast agent (magnevist, produced by Bayer Corp.) is first injected through tail vein as reference (injection dosage: 0.1 mmol/kg) and is scanned continuously for 2 hours in the magnetic resonance chamber. After the Gd-DTPA is completely metabolized and excreted, human H-subunit magneto ferritin is injected through tail veil (injection dosage: 20 mg Fe/kg mouse weight) and is scanned continuously for 3 hours. And scanning is conducted again after 24 hours in order to exclude the interference of blood. MSME T₁-weighted imaging is used for the injected Gd-DTPA, and the parameters used are as follows: FOV = 2 cm × 2 cm, matrix = 128 × 128, TR = 3000 ms, TE = 350 ms, 20 slices, slice thickness 0.80 mm. For the injected magnetoferritin, T₂-weighted imaging uses MSME sequence and T₂*-weighted imaging uses MGE sequence. The sequence parameters are substantially similar to those of embodiment 19, matrix : 128 × 128.

FIGS. 23a-23c are the magnetic resonance scanning photos prior to and after Gd-DTPA injection. It can be seen that the signal intensity of the glioma area shows no evident change after injecting the commercialized Gd-DTPA. FIGS. 23d-f are the T₂-weighted magnetic resonance imaging photos (echo time 32 ms) prior to and after magnetoferritin injection. FIGS. 23g-23i are the T₂*-weighted magnetic resonance imaging photos (echo time 4.5 ms). FIGS. 23j-23l are the second T₂*-weighted magnetic resonance imaging photos (echo time 12 ms). Both the T₂-weighted photos and the T₂*-weighted photos show clearly that after magnetoferritin injection, evident changes visible to the naked eye appear in the glioma area with a size of about 1 mm. After 24 hours, interference of blood is excluded. The tumor still clearly shows a low signal intensity. The signal intensity is notably lower than prior to injection. The tumor shows a good tissue contrast against surrounding normal tissues and the tumor tissues can be clearly distinguished. In order to verify the detection result of the magnetic resonance imaging, after the magnetic resonance scanning conducted 24 hours after injection, heart PBS and 4% paraformaldehyde infusion are conducted on the nude mouse. The cerebral tissues are extracted completely and dehydrated using 10%, 20% and 30% sucrose, respectively. The tissues are then encapsulated with OCT and transversely sliced (slice thickness 20 µm) on a frozen tissue slicer. H&E staining is conducted for the identification of tumor tissues; DAB-enhanced Prussian blue staining is used to stain iron particles; and anti-TfR1 antibodies are used to stain TfR1 expression.

### Embodiment 24

### Magnetoferritin used in early MRI diagnosis of in-situ pancreatic cancer

By cutting open the left flank pancreas spot on the abdominal cavity of the nude mouse, 10⁶ CFPAC-1 pancreatic carcinoma cells (purchased from ATCC, cultivated at Nanjing KeyGEN BioTECH development Ltd.) are in-situ inoculated onto the pancreatic tissues beneath the spleen of the nude mouse. After about 3∼4 days, human H-subunit magnetoferritins are injected, and T₂-weighted and T₂^{*}-weighted magnetic resonance imaging are conducted. FIGS. 24a-24b and FIGS. 24c-24d are the T₂-weighted and T₂^{*}-weighted magnetic resonance imaging photos of the in-situ pancreatic carcinoma implanted nude mouse model prior to and after magnetoferritin injection, respectively. Both the T₂-weighted photos and the T₂^{*}-weighted photos show clearly that 24 hours after magnetoferritin injection, evident changes visible to the naked eye appear in the in-situ pancreatic carcinoma area with a size of about 1 mm and can be clearly distinguished from surrounding normal tissues. 24 hours after injection and after magnetic resonance scanning, the mouse is sacrificed by cervical dislocation. The abdominal cavity of the mouse is cut open in prone position. All the tissues and organs in the abdominal cavity are removed along the esophagus to expose the spleen and the pancreas for the observation of position where the tumor grows on the pancreas (FIG. 24e). In order to further verify the position of the tumor tissues, the spleen and pancreas tissues are extracted together, fixed using 4% paraformaldehyde, dehydrated using 10%, 20% and 30% sucrose, and encapsulated with OCT and transversely sliced (slice thickness 20 µm) on a frozen tissue slicer. H&E staining is conducted for the identification of tumor tissues; DAB-enhanced Prussian blue staining is used to stain iron particles; and anti-TfR1 antibodies are used to stain TfR1 expression.

### Embodiment 25

### Biomimetic synthesis of ferritin-gadolinium-iron dual-mode magnetic resonance contrast agent composite

Gd contrast agents in clinical use can enhance the signal intensity of the lesion area during T₁-weighted magnetic resonance imaging, thus showing a bright area. Since human naked eye is more sensitive to bright matters, these contrast agents can better meet the needs of human naked eye. But Gd contrast agents in clinical use have no targeting feature and have a low relaxation rate, thus failing to meet the needs for early diagnosis of diseases such as tumors. In the present invention, a tumor-targeted ferritin shell is used, and in the cavity thereof a gadolinium-iron nano-material is formed by biomimetic mineralization. By using purified human H-subunit ferritin as a template, a ferrous salt, a gadolinium salt and oxidant hydrogen peroxide are added into a solution of recombinant human ferritin, controlling the pH at 8.5 and the temperature at 65°C. By precisely controlling the proportion relation between ferrous ions, gadolinium particles and proteins, and by precisely controlling the temperature and pH to be constant, the final number of iron atoms added into a single protein molecule is about 150, and the theoretical proportion of gadolinium added is about 3%. After the reaction is completed, mono-dispersed ferritin-gadolinium iron oxide nano-particles with a complete protein structure are obtained through size-exclusion chromatography, centrifugation and molecular sieve purification. The numbers of iron atoms and gadolinium atoms are measured through inductively coupled plasma mass spectrometry. The longitudinal relaxation time (r₁) and transverse relaxation time (r₂) of the material are measured using a 4.7T magnetic resonance imaging system.

Through the ICP-MS measurement, an average of about 1140 iron atoms and 31 gadolinium atoms are loaded into a single protein shell.

Fig. 25a shows the T₁-weighted magnetic resonance imaging photos (echo time TE = 8.5 ms, reversal time TR = 300 ms) of commercialized Gd-DTPA (magnevist, produced by Bayer Corp.) with various gadolinium concentrations (0-1 mM). Fig. 25b shows the linear relation between the reciprocals of the measured longitudinal relaxation time (R₁ = 1/T₁) of Gd-DTPA with various gadolinium concentrations. It can be deduced from the figure that the longitudinal relaxation rate r₁ in PBS solution is about 5.9 mM⁻¹S⁻¹ at the field intensity of 4.7T. Fig. 25c shows the T₁-weighted imaging photos (TE = 8.5 ms; TR = 300 ms) of ferritin-gadolinium-iron composite various gadolinium concentrations (0-1 mM). Fig. 25d shows the linear relation between the reciprocals of the measured longitudinal relaxation time (R₁ = 1/T₁) of ferritin-gadolinium-iron composite with various gadolinium concentrations. It can be deduced from the figure that the longitudinal relaxation rate r₁ is about 4.1 mM⁻¹S⁻¹. Fig. 25e shows the T₂-weighted imaging photos for various iron concentrations. It can be seen from the figure that ferritin-gadolinium-iron composite not only has the function of gadolinium T₁ contrast agent, that is, enhancing the signal intensity of T₁-weighted imaging to show bright area in the image, but also has the function of iron oxide T₂ contrast agent, that is, weakening the signal intensity to show dark area. Fig. 25f shows the linear relation between the reciprocals of the measured transverse relaxation time (R₂ = 1/T₂) of ferritin-gadolinium-iron composite with various iron concentrations. It can be deduced from the figure that the transverse relaxation rate r₁ is about 9.0 mM⁻¹S⁻¹.

### Embodiment 26

### Ferritin-gadolinium-iron dual-mode magnetic resonance contrast agent composite used in specific early diagnosis of in-situ hepatocellular carcinoma

By cutting open the abdominal cavity of a nude mouse along the abdominal median line, 10⁶ QGY7701 human hepatocellular carcinoma cells (purchased from ATCC, cultivated at Nanjing KeyGEN BioTECH development Ltd.) are in-situ inoculated inside the hepatic tissue capsule on the left flank of the nude mouse. After about 3-4 days, T₁-weighted, T₂-weighted and T₂^{*}-weighted magnetic resonance imaging are conducted. Fig. 26a-26b show the T₁-weighted magnetic resonance imaging photos of the in-situ hepatocellular carcinoma nude mouse transplantation model prior to and after the injection of ferritin-gadolinium-iron dual-mode magnetic resonance contrast agent. It can be seen that the signal intensity of the tumor area is enhanced, showing a bright area. Fig. 26c-26d and Fig. 26e-26f show the T₂-weighted and T₂^{*}-weighted magnetic resonance imaging photos of the in-situ hepatocellular carcinoma nude mouse transplantation model prior to and after the injection of ferritin-gadolinium-iron dual-mode magnetic resonance contrast agent. Both the T₂-weighted photos and the T₂^{*}-weighted photos show clearly that 96 hours after magneto ferritin injection, evident changes visible to the naked eye appear in the in-situ hepatocellular carcinoma area with a size of about 5 mm and can be clearly distinguished from surrounding normal tissues.

### Embodiment 27

### Ferritin-gadolinium-iron dual-mode magnetic resonance contrast agent composite used in specific early diagnosis of in-situ glioma

10⁶ U87MG human glioma cells (purchased from ATCC, cultivated at Nanjing KeyGEN BioTECH development Ltd.) are inoculated onto the cerebral cortex of a nude mouse by in-situ penetration through the cranium and its tympanic membrane slightly off the median line of the cerebrum. Magnetic resonance imaging is conducted after about 3-4 days. The nude mouse is anesthetized by respiratory anesthesia and an intravenous needle is detained in tail vein. The mouse is put into a 4.7T MRI system and remains in stationary state in the course of magnetic resonance scanning. T₁-weighted and T₂^{*}-weighted magnetic resonance imaging are conducted. Fig. 27a-27b show the T₁-weighted magnetic resonance imaging photos of the in-situ hepatocellular carcinoma nude mouse transplantation model prior to and after the injection of ferritin-gadolinium-iron dual-mode magnetic resonance contrast agent. The signal intensity of the tumor area is slightly enhanced, showing a bright area. Fig. 27c-27d show the T₂^{*}-weighted magnetic resonance imaging photos of the in-situ glioma nude mouse transplantation model prior to and after the injection of ferritin-gadolinium-iron dual-mode magnetic resonance contrast agent. The T₂^{*}-weighted photo clearly shows that 24 hours after the injection of ferritin-gadolinium-iron dual-mode magnetic resonance contrast agent, evident changes visible to the naked eye appear in the in-situ glioma area with a size of about 2 mm with notably lowered signal intensity and can be clearly distinguished from surrounding normal tissues.

### Embodiment 28

### Synthesis of ferritin coated Gd-DTPA composite and its use in early diagnosis of in-situ glioma

Purified human H-subunit ferritin is partially or totally denatured using denaturant such as guanidine hydrochloride (1-7 M) or urea (2-8 M) etc. such that the subunits are partially or totally depolymerized. About 2000 commercialized Gd-DTPA gadolinium atoms are added into a single protein molecule. Then dialysis is conducted to remove the denaturant such that the subunits re-polymerize to form a cage structure and encapsulate Gd-DTPA into the cavity of the protein. The unrenatured ferritins are removed using centrifugation, molecular sieve chromatography or anion exchange. Protein quantification is conducted using BCA protein quantification reagent kit. The number of Gd atoms encapsulated in a single protein molecule is measured using ICP-MS. Through the ICP-MS measurement, about 18 Gd-DTPA molecules are encapsulated in a single protein molecule. U87MG human glioma cells are in-situ inoculated onto the cerebral cortex of a nude mouse to establish a nude mouse glioma in-situ model. Magnetic resonance imaging is conducted after about 3-4 days. The nude mouse is anesthetized by respiratory anesthesia and an intravenous needle is detained in tail vein. The mouse is put into a 4.7T MRI system and remains in stationary state in the course of magnetic resonance scanning. Human H-subunit ferritin coated Gd-DTPA composite is injected through tail vein (injection dosage quantified according to Gd quantity, i.e., 0.1 mmol/kg) and scanned continuously for 2 hours. Then it is scanned again after 24 hours to exclude the interference of blood.

Fig. 28a is the schematic chart for the process of human H-subunit ferritin encapsulating Gd-DTPA. Fig. 28b-28d show the T₁-weighted magnetic resonance imaging photos prior to and after the injection of human H-subunit ferritin coated Gd-DTPA composite. The photos show clearly that 24 hours after the injection of magneto ferritin, evident changes visible to the naked eye appear in the glioma area with a size of about 1 mm, showing high signal intensity area (bright area), and can be clearly distinguished from surrounding normal tissues.

All literature mentioned in the present invention are cited as reference in the present application, as every reference is separately cited. Herein it shall be construed that after reading the above teachings of the present invention, a person skilled in the art can make various alterations and modifications to the present invention, and these equivalents fall into the scope limited by the claims of the present application.

## Claims

1. Use of a protein shell coated magnetic nano-particle or a derivative thereof for the manufacture of an image localization diagnostic reagent and a therapeutic agent.

2. The use of claim 1, **characterized in that** said image localization diagnostic reagent is selected from a magnetic resonance imaging reagent or a molecular probe.

3. The use of claim 2, **characterized in that** said magnetic resonance imaging reagent or the molecular probe comprises said protein shell coated magnetic nano-particles or derivatives thereof.

4. The use of claim 1, wherein the shell coated magnetic nano-particle or the derivative thereof is **characterized in that** a core of the magnetic nano-particle or the derivative thereof comprises a metal compound comprising a metal element, wherein said metal element is selected from gadolinium, manganese, iron, cobalt and/or nickel.

5. The use of claim 1, **characterized in that** said protein shell of the protein shell coated magnetic nano-particle or the derivative thereof can bind specifically to a receptor expressed on a surface of a tissue or a cell.

6. The use of claim 5, **characterized in that** said protein shell is selected from a ferritin, a chaperone protein, a DNA binding protein, a magnetosome membrane protein of a magnetotactic bacteria, or a viral protein shell having a nano-cavity structure.

7. The use of claim 6, **characterized in that** said ferritin comprises a natural ferritin or a genetically engineered recombinant ferritin, wherein the natural ferritin is from an eukaryote or a prokaryote, and wherein the genetically engineered recombinant ferritin is a recombinant ferritin that comprises a heavy (H) chain subunit, a recombinant ferritin that comprises a light (L) chain subunit, a recombinant ferritin that is assembled from the heavy chain and light chain subunits in any proportion, or a mutant or a fusion protein of said protein subunits.

8. The use of claim 1, **characterized in that** said the protein shell coated magnetic nano-particle or the derivative thereof is prepared by the following steps:
(a) based on a recombinant human ferritin, cloning a full length cDNA of the H-subunit and a full length cDNA of the L-subunit, and constructing the full length cDNA of the H-subunit and the full length cDNA of the L-subunit separately into pET11b plasmids;
(b) transfecting, separately or cotransfecting, BL21(DE3)plysS cells with the recombinant plasmids comprising the human ferritin H-subunit and L-subunit, and then adding isopropyl-β-D-thiogalactopyranoside to activate T7 promoter and induce expression;
(c) releasing expressed proteins by breaking the cells using ultrasound after protein expression;
(d) isolating and purifying a recombinant human ferritin;
(e) adding a metal salt that forms a composition of the core and an oxidant to a solution of the recombinant human ferritin to undergo reaction, controlling pH at 7-11 and a temperature at 25-80°C, to form strongly magnetic nano-particles within the recombinant human ferritin, wherein a concentration of the salt that forms the composition of the core is such that a ratio of the number of metal atoms to the number of protein molecules is between 10-200, so that the number of metal atoms added in a single protein molecule is between 100-15000; a concentration of the oxidant is such that a ratio of the number of oxidant molecules to the number of added metal ions is 2:1 or 3:1; and a concentration of the recombinant human ferritin is greater than 0.25mg/mL; and
(f) obtaining the protein shell coated magnetic nano-particle or derivative thereof after isolation by size exclusion or ion-exchange chromatography, and purification by centrifugation and molecular sieve or anion-exchange chromatography.

9. The use of claim 8, **characterized in that** the pH is controlled at 8-9 in step (e).

10. The use of claim 8, **characterized in that** the temperature is controlled at 35-70°C in step (e).

11. The use of claim 8, **characterized in that** said metal salt forming the composition of the core is selected from a ferrous salt, a ferric salt, a gadolinium salt, a manganese salt, a cobalt salt and/or a nickel salt.

12. The use of claim 8, **characterized in that** said metal element is selected from gadolinium, manganese, iron, cobalt and/or nickel.

13. The use of claim 8, **characterized in that** said oxidant is selected from hydrogen peroxide, oxygen gas, or a substance that can produce hydrogen peroxide or oxygen in a reaction.

14. The use of claim 8, **characterized in that** the number of the metal atoms added into a single protein molecule is between 140-10000.

15. The use of claim 8, **characterized in that** the number of gadolinium atoms added into a single protein molecule is between 100-200, and/or the number of iron atoms added into a single protein molecule is between 500-10000.

16. The use of claim 1, **characterized in that** said therapeutic agent is a substance for treating a disease associated with ferritin receptor expression.

17. The use of claim 16, **characterized in that**, said therapeutic agent is linked with the protein shell that encapsulates the magnetic nano-particle.

18. The use of claim 16, **characterized in that** said therapeutic agent is selected from a chemotherapeutic agent, a radioactive isotope, a cytokine, a nucleic acid, and an anti-cancer or anti-inflammation agent.

19. The use of any one of claims 16-18, **characterized in that** said disease associated with ferritin receptor expression is tumor and/or inflammation.

20. The use of claim 19, **characterized in that** said tumor is selected from hepatocellular carcinoma, leukemic carcinoma, glioma, pulmonary carcinoma, colonic carcinoma, pancreatic carcinoma, or mammary carcinoma.

21. Use of a protein shell coated magnetic nano-particle or a derivative thereof for the manufacture of a magnetic contrast agent and a molecular probe for diagnosis of a disease associated with ferritin receptor expression.

22. The use of claim 21, **characterized in that** said disease associated with ferritin receptor expression is tumor and/or inflammation.

23. The use of claim 22, **characterized in that**, said tumor is selected from mammary carcinoma, hepatocellular carcinoma, pulmonary carcinoma, colonic carcinoma, pancreatic carcinoma, glioma, leukemic carcinoma, or prostatic carcinoma.

24. A protein shell coated magnetic nano-particle or a derivative thereof, **characterized in that** a composition of a core of said magnetic nano-particle or the derivative thereof is a compound that comprises a metal element, said metal element is selected from gadolinium, manganese, iron, cobalt, and/or nickel.

25. The protein shell coated magnetic nano-particle or the derivative thereof according to claim 24, **characterized in that** the composition of the core of said protein shell coated magnetic nano-particle or the derivative thereof is a compound comprising iron.

26. The protein shell coated magnetic nano-particle or the derivative thereof according to claim 24, **characterized in that** the composition of the core of said protein shell coated magnetic nano-particle or the derivative thereof is a compound comprising gadolinium.

27. The protein shell coated magnetic nano-particle or the derivative thereof according to claim 24, **characterized in that** the composition of the core of said protein shell coated magnetic nano-particle or the derivative thereof is a compound comprising iron and manganese.

28. The protein shell coated magnetic nano-particle or the derivative thereof according to claim 24, **characterized in that** the composition of the core of said protein shell coated magnetic nano-particle or the derivative thereof is a compound comprising iron and gadolinium.

29. The protein shell coated magnetic nano-particle or the derivative thereof according to any of claims 24-28, **characterized in that** said protein shell is selected from a ferritin, a chaperone protein, a DNA binding protein, a magnetosome membrane protein of magnetotactic bacteria or a virus protein shell having a nano-cavity structure; said protein shell of the protein shell coated magnetic nano-particle or the derivative thereof can bind specifically to a receptor expressed a the surface of a tissue or cell.

30. The protein shell coated magnetic nano-particle or the derivative thereof according to claim 29, **characterized in that** said ferritin includes a natural ferritin or a genetically engineered recombinant ferritin, wherein the natural ferritin is from an eukaryote or a prokaryote, and wherein the genetically engineered recombinant ferritin is a recombinant ferritin that comprises a heavy (H) chain subunit, a recombinant ferritin that comprises a light (L) chain subunit, a recombinant ferritin that is assembled from the heavy chain and light chain subunits in any proportion, or a mutant or a fusion protein of said protein subunits.

31. A method for preparing said protein shell coated magnetic nano-particle or the derivative thereof according to any one of claims 24-30, **characterized in that** said method comprises the following steps:
(a) based on a recombinant human ferritin, cloning a full length cDNA of the light (L) chain subunit and a full length cDNA of the L-subunit, and constructing the full length cDNA of the H-subunit and the full length cDNA of the light (L) chain subunit separately into pET11b plasmids;
(b) transfecting, separately or cotransfecting, BL21(DE3)plysS cells with the recombinant plasmids comprising the human ferritin H-subunit and L-subunit, and then adding isopropyl-β-D-thiogalactopyranoside to activate T7 promoter and induce expression;
(c) releasing expressed proteins by breaking the cells using ultrasound after protein expression;
(d) isolating and purifying a recombinant human ferritin;
(e) adding a metal salt that forms a composition of the core and an oxidant to a solution of the recombinant human ferritin to undergo reaction, controlling pH at 7-11 and a temperature at 25-80°C, to form strongly magnetic nano-particles within the recombinant human ferritin, wherein a concentration of the salt that forms the composition of the core is such that a ratio of the number of metal atoms to the number of protein molecules is between 10-200, so that the number of metal atoms added in a single protein molecule is between 100-15000; a concentration of the oxidant is such that a ratio of the number of oxidant molecules to the number of added metal ions is 2:1 or 3:1; and a concentration of the recombinant human ferritin is greater than 0.25mg/mL; and
(f) obtaining the protein shell coated magnetic nano-particle or derivative thereof after isolation by size exclusion or ion-exchange chromatography, and purification by centrifugation and molecular sieve or anion-exchange chromatography.

32. The method of claim 31, **characterized in that** the pH is controlled at 8-9 in step (e).

33. The method of claim 31, **characterized in that** the temperature is controlled at 35-70°C in step (e).

34. The method of claim 31, **characterized in that** said metal salt forming the composition of the core is selected from a ferrous salt, a ferric salt, a gadolinium salt, a manganese salt, a cobalt salt, and/or a nickel salt.

35. The method of claim 31, **characterized in that** said metal element is selected from gadolinium, manganese, iron, cobalt, and/or nickel.

36. The method of claim 35, **characterized in that** said metal element is iron.

37. The method of claim 35, **characterized in that** said metal element is manganese.

38. The method of claim 35, **characterized in that** said metal element is gadolinium.

39. The method of claim 35, **characterized in that** said metal element is manganese and iron.

40. The method of claim 35, **characterized in that** said metal element is gadolinium and iron.

41. The method of claim 31, **characterized in that** said oxidant is selected from hydrogen peroxide, oxygen gas, and a substance that can produce hydrogen peroxide or oxygen gas in a reaction.

42. The method of claim 31, **characterized in that** the number of the metal atoms added into a single protein molecule is between 140-10000.

43. The method of claim 31, **characterized in that** the number of gadolinium atoms added into a single protein molecule is between 100-200, and/or the number of iron atoms added into a single protein molecule is between 500-10000.
